# EUROPEAN PATENT SPECIFICATION

(11) **EP 4 058 059 B1**
(45) Date of publication and mention of the grant of the patent: **09.10.2024**
(21) Application number: 20888060.9
(22) Date of filing: 13.11.2020
(51) Int. Cl.: A61K 9/107, A61K 39/35, A61K 39/36, A61K 9/113, A61P 37/08, B82Y 5/00

(54) **NANOEMULSION COMPOSITIONS FOR TREATING AEROALLERGEN ASSOCIATED ALLERGY AND INFLAMMATION**
NANOEMULSIONSZUSAMMENSETZUNGEN ZUR BEHANDLUNG VON MIT AEROALLERGENEN VERBUNDENEN ALLERGIEN UND ENTZÜNDUNGEN
COMPOSITIONS DE NANO-ÉMULSION POUR LE TRAITEMENT D'UNE ALLERGIE ET D'UNE INFLAMMATION ASSOCIÉES À DES AÉROALLERGÈNES

(30) Priority: 13.11.2019 US 201962934878 P
(43) Date of publication of application: 21.09.2022
(73) Proprietor: The Regents Of The University Of Michigan, Ann Arbor, Michigan 48109 (US)
(72) Inventor: LUKACS, Nicholas, ANN ARBOR, Michigan 48109-2590 (US); BAKER, JAMES R., Jr., ANN ARBOR, Michigan 48109-2590 (US); O'KONEK, Jessica, ANN ARBOR, Michigan 48109-2590 (US)
(74) Representative: Algemeen Octrooi- en Merkenbureau B.V.
(86) International application number: PCT/US2020/060362
(87) International publication number: WO 2021/097174

(56) References cited:
- US-A1- 2006 216 315
- US-A1- 2017 246 294
- US-A1- 2017 246 294
- MEECHAN PANISSARA ET AL: "Intranasal, liposome-adjuvanted cockroach allergy vaccines made of refined major allergen and whole-body extract of Periplaneta americana", INTERNATIONAL ARCHIVES OF ALLERGY AND IMMUNOLOGY, S. KARGER AG, CH, vol. 161, no. 4, 1 January 2013 (2013-01-01), pages 351 - 362, XP009544289, ISSN: 1423-0097, DOI: 10.1159/000348314

## Description

### STATEMENT REGARDING FEDERALLY SPONSORED RESEARCH AND

### DEVELOPMENT

This invention was made with Government support under Grant Number AI036302 awarded by the National Institute of Allergy and Infectious Diseases (NIAID). The Government has certain rights in this invention.

### CROSS-REFERENCE TO RELATED APPLICATIONS

This application claims the benefit of U.S. Provisional Patent Application No. 62/938,878, filed November 13, 2019.

### FIELD

This disclosure relates to compositions and methods for treating aeroallergen induced inflammation (e.g., airway inflammation) or other adverse condition (e.g., allergic condition).

The references to the methods of treatment by therapy are to be interpreted as references to pharmaceutical compositions of the present invention for use in those methods

### BACKGROUND

Allergic diseases caused by airborne allergens such as allergic rhinitis and respiratory allergies (e.g., pollen allergies) are serious health issues in the United States and in other countries. In particular, allergic reactions to substances such as pollens (hay fever) or other plant components, cat fur and other animal hairs, dust and dust mite droppings, or perfumes and other components of cosmetics, are a growing problem for increasing numbers of people. Overall, allergic diseases are among the major causes of illness and disability in the United States, affecting more than 50 million Americans annually, and allergies are the 6^{th} leading cause of chronic illness in the U.S (U.S. Centers for Disease Control and Prevention (CDC), Allergies: Gateway to Health Communication (CDC)).

Conventional treatment for allergic diseases caused by airborne allergens include medications such as steroids or other types of drugs that inhibit the release of inflammatory substances in mast cells. Such treatments, however, often trigger a number of undesirable side effects, do not address the underlying immunological bases of the disease, and must be continually taken by a patient in order to provide a benefit.

There remains a need for compositions and methods that effectively treat allergies, particularly allergies induced by airborne allergens ("aeroallergens").

Cockroach allergy vaccines are disclosed in US 2006/216315, and MEECHAN PANISSARA ET AL: "Intranasal, liposome-adjuvanted cockroach allergy vaccines made of refined major allergen and whole-body extract of Periplaneta americana.

US 2017/246294 describes the use of a nanoemulsion as adjuvant for the preparation of vaccine for preventing or treating allergies and inflammatory diseases. It does not describe cockroach allergen.

### BRIEF SUMMARY OF THE INVENTION

The disclosure provides a composition comprising a nanoemulsion and one or more aeroallergens wherein the aeroallergen is a cockroach allergen, and wherein the nanoemulsion comprises:(a) a poloxamer surfactant or polysorbate surfactant; (b) an organic solvent;(c) a halogen-containing compounded) oil, and(e) water. The disclosure also provides the treatment of (e.g., therapeutically or prophylactically) aeroallergen induced inflammation (e.g., airway inflammation) in a subject, which comprises administering an effective amount of the aforementioned composition to a subject in need thereof, whereupon the aeroallergen induced inflammation (e.g., airway inflammation) in the subject is treated.

The invention is defined in the appended claims. Any disclosure going beyond the scope of said claims is only intended for illustrative purposes,

Any references in the description to methods of treatment refer to the compounds, pharmaceutical compositions and medicaments of the present invention for use in a method for treatment of the human (or animal) body by therapy.

### BRIEF DESCRIPTION OF THE DRAWING(S)

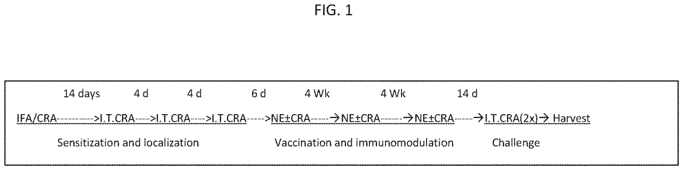

FIG. 1 is a diagram illustrating the sensitization and challenge schedule for development of allergen-induced airway disease model in mice. Fourteen days after the original immunization to cockroach allergen (CRA), mice were given intratracheal (IT) administration of allergen 2x every four days. At day 28, six days after final IT sensitization, animals received the nanoemulsion vaccine (NE) with or without CRA followed by two more immunizations with the NE and CRA at four-week intervals by intranasal application. After a final 14 days, animals were challenged with allergen by IT administration twice, four days apart and examined for allergic responses 24 hours after the final IT challenge.

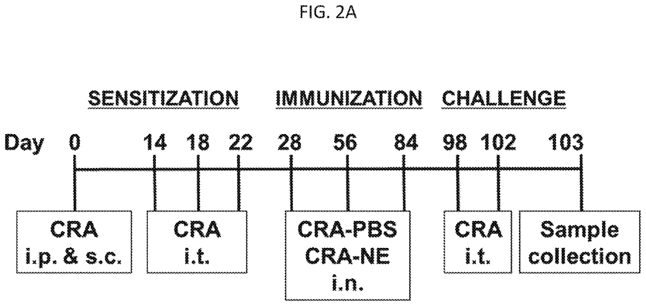

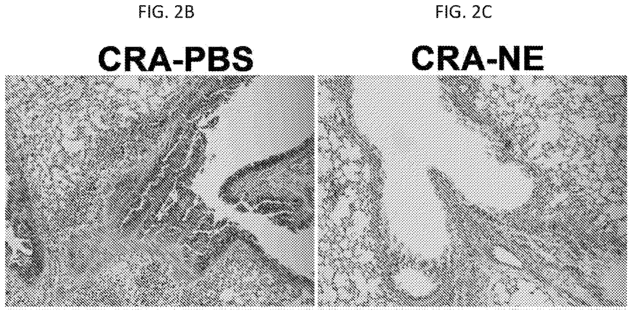

FIG. 2A is a schematic diagram illustrating the schedule of sensitization, immunotherapy, and allergen. FIGS. 2B and 2C are representative images of lungs from mice treated with CRA only (FIG. 2B) or nanoemulsion plus CRA (FIG. 2C). Lungs were stained with Periodic acid Schiff (PAS), which stains mucus red-pink.

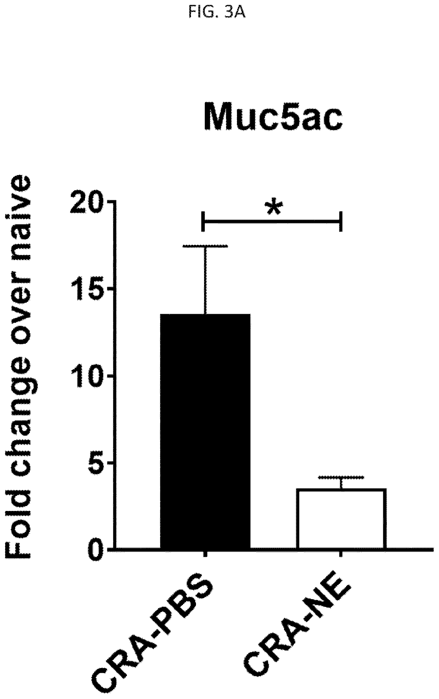

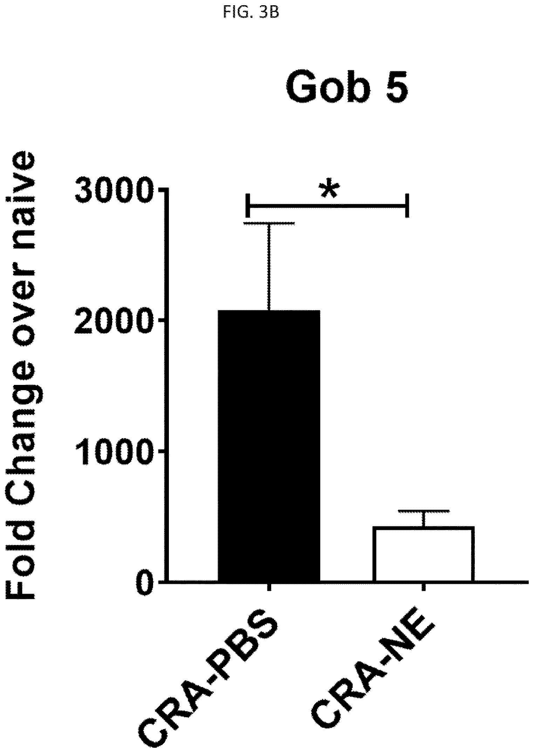

FIGS. 3A and 3B are graphs illustrating quantitative PCR of lung mRNA (Muc5ac: FIG. 3A; Gob 5: FIG. 3B) that was isolated from individual mice in order to analyze the critical disease-associated mRNA compared to age-matched naive, non-allergic mice.

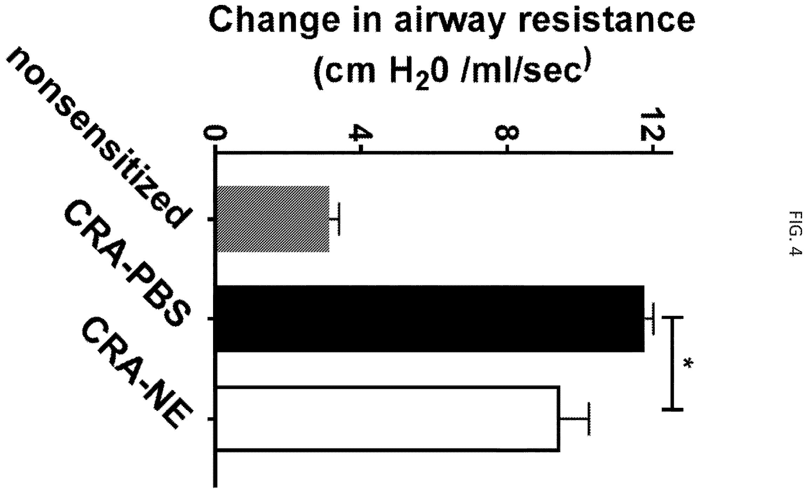

FIG. 4 is a graph illustrating airway resistance (AHR) measurements in animals 24 hours after final allergen challenge using plethysmography. An IV tail vein injection of methacholine (250 µg/kg) was used to induce AHR. Data are expressed as mean ± standard deviation (n=8). Statistically significant differences (p<0.05) are indicated by *.

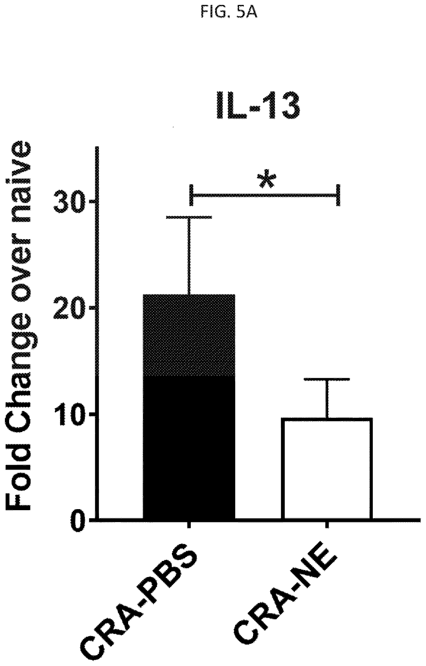

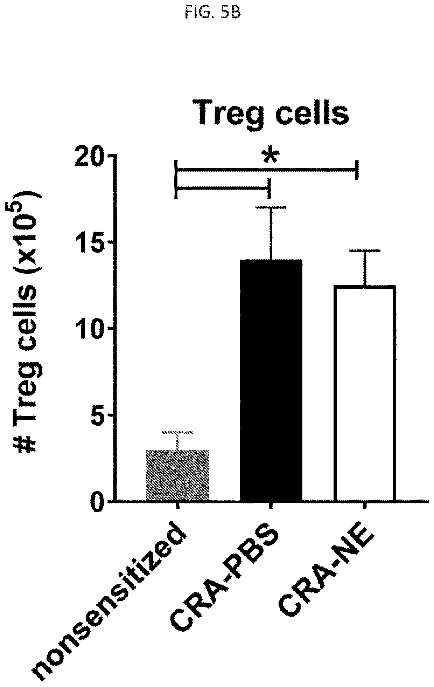

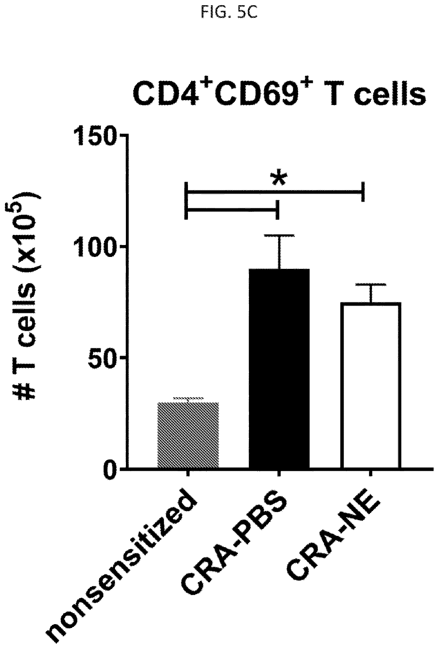

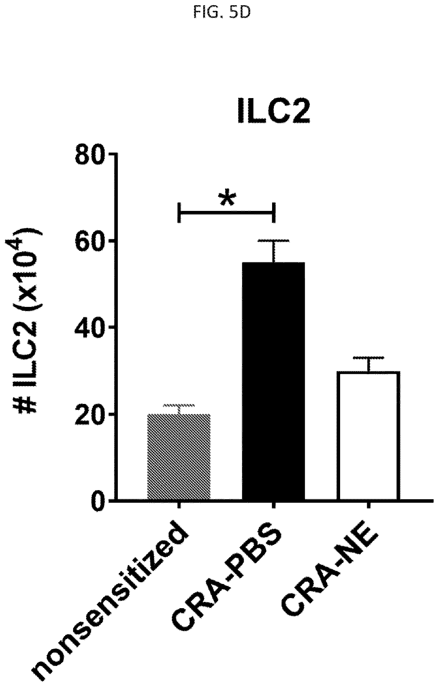

FIGS. 5A-5D are graphs illustrating flow cytometry results of lung cells from naïve or allergen-challenged mice. Lung mRNA was isolated from individual mice and subjected to quantitative PCR to analyze the mucus associated mRNA compared to age matched naive, non-allergic mice. Lungs from allergen challenged mice were dispersed into a single cell suspension using collagenase digestion. FIG. 5A shows that IL-13 significantly decreased in the lungs of mice that received the NE vaccine before antigen challenge. Treg cells (CD4⁺, CD25⁺, Foxp3⁺) (FIG. 5B), activated Th cells (CD4⁺ CD69⁺) (FIG. 5C), and ILC2 cells (Lin⁻, CD45⁺, CD90⁺, ST2⁺, GATA3⁺) (FIG. 5D) were incubated with fluorescent antibodies for cellular identification by flow cytometry. Data are expressed as mean ± standard deviation (n=8). Statistically significant differences (p<0.05) are indicated by *.

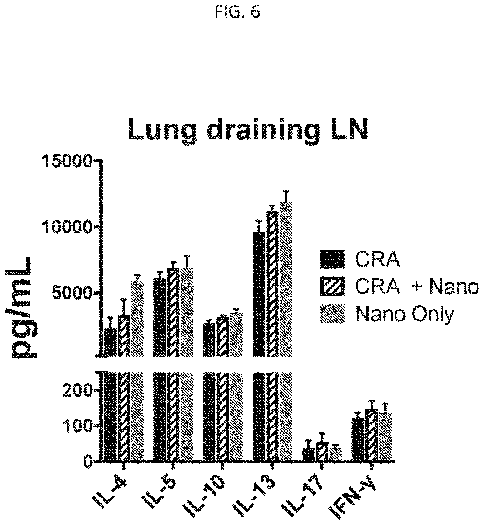

FIG. 6 is a graph illustrating results of a cytokine activation assay. Lung draining lymph nodes (LN) were harvested at the end of the response and single cell suspensions rechallenged with allergen. Data represents mean ± SE from 7-8 mice/group.

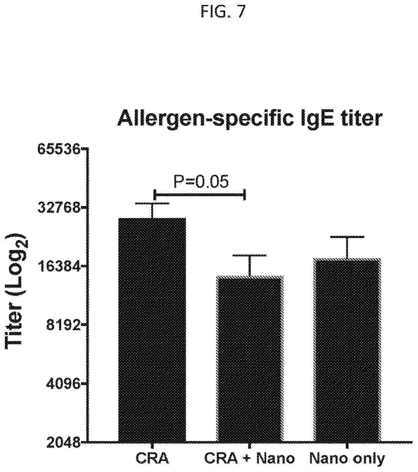

FIG. 7 is a graph showing allergen-specific IgE titers in treated mice. Serum was collected from mice at the end of the challenge protocol and subjected to allergen-specific IgE ELISAs and recorded as the highest titer that gave a positive signal. 7-8 mice/group.

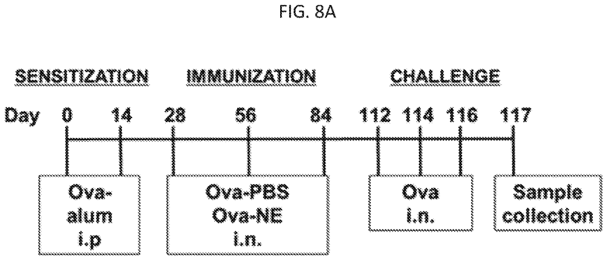

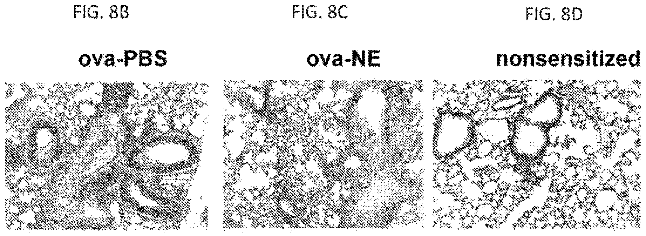

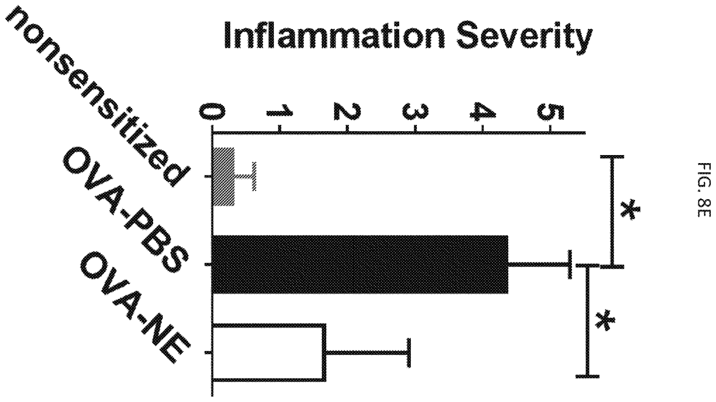

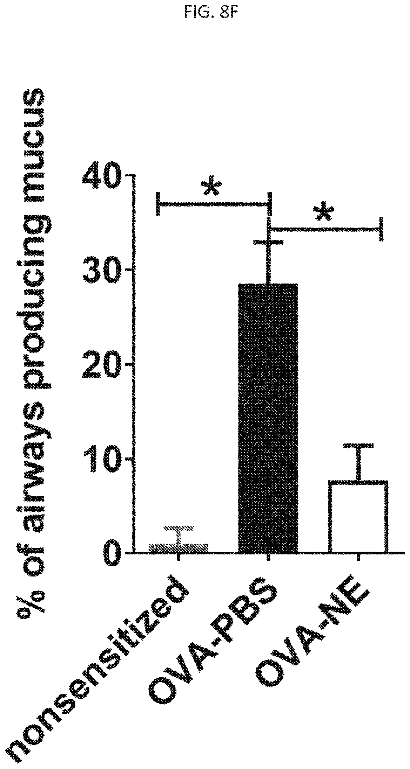

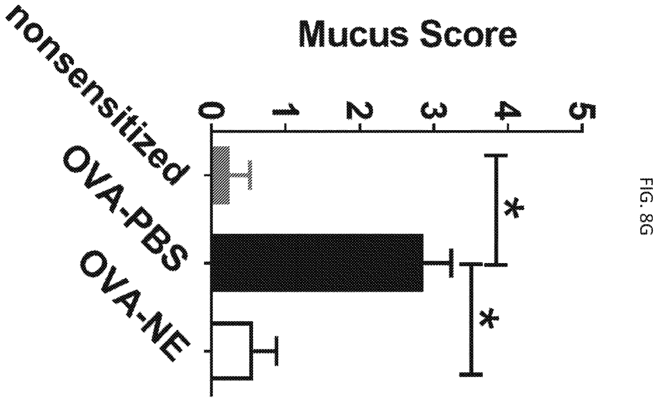

FIG. 8A is a schematic diagram illustrating the schedule of sensitization, immunotherapy, and allergen challenge described in Example 3. Mice were sacrificed two days after the last challenge to assess lung histopathology. FIGS. 8B, 8C, and 8D are representative images of PAS staining of lungs. FIG. 8E is a graph showing scoring of severity of lung inflammation. FIG. 8F is a graph showing scoring of severity of lung mucus. FIG. 8G is a graph of mucus scores. Data are expressed as mean ± standard deviation (n=8). Statistically significant differences (p<0.05) are indicated by *.

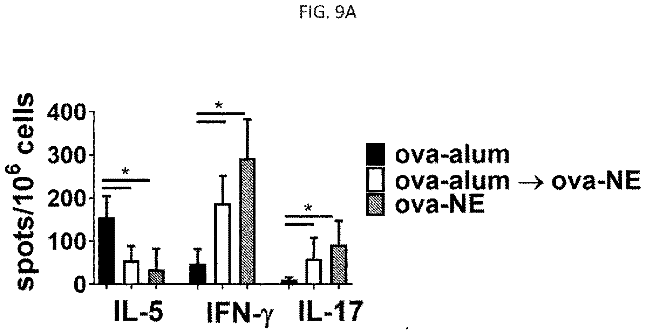

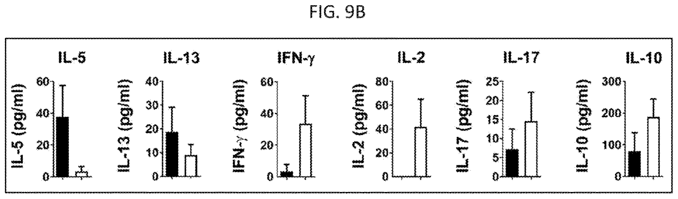

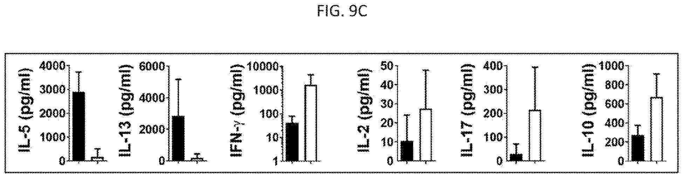

FIG. 9A is a graph showing numbers of cytokine producing cells in cervical lymph nodes (cLN) as determined by ELISpot. FIGS. 9B and 9C are graphs showing cytokine secretion in both cLN (FIG. 9B) and splenocyte (FIG. 9C) cultures as determined by a Luminex multiplex assay. Data are expressed as mean ± standard deviation (n=8). Statistically significant differences 571 (p<0.05) are indicated by *.

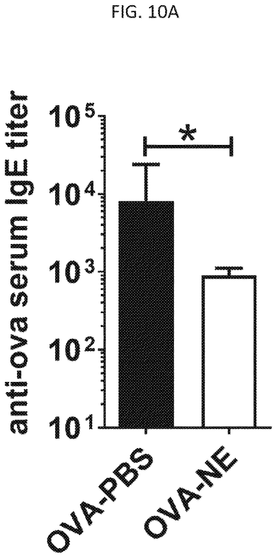

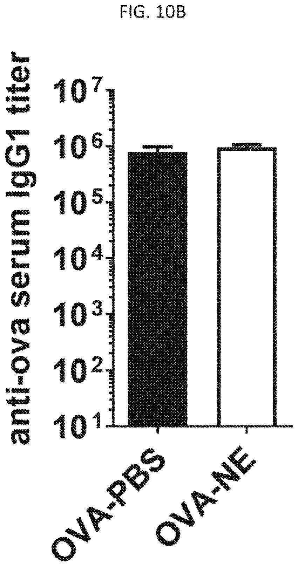

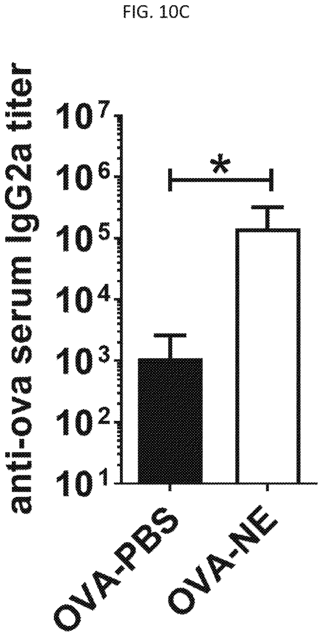

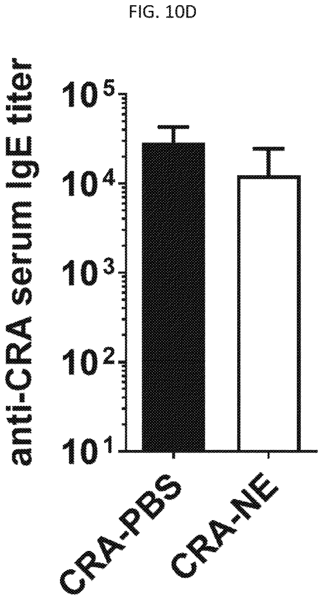

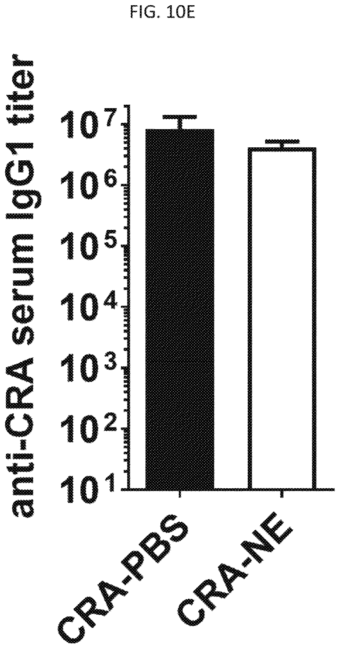

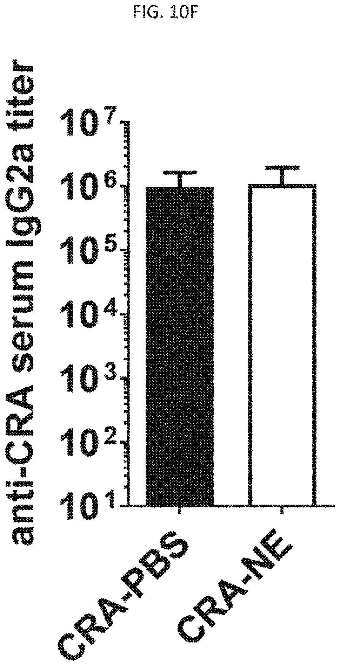

FIGS. 10A-10F are graphs illustrating the persistence of allergen-specific IgE in serum obtained from mice at the end of the study in the ova asthma model and the chronic CRA asthma model. Ova-specific IgE, IgG1, and IgG2a antibodies measured by ELISA are showing FIGS. 10A, 10B, and 10C, respectively. CRA-specific IgE, IgG1 and IgG2a antibodies measured by ELISA are shown in FIGS. 10D, 10E, and 10F. Statistically significant differences (p<0.05) are indicated by *.

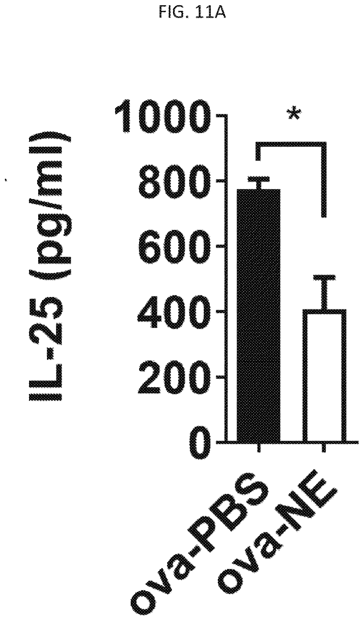

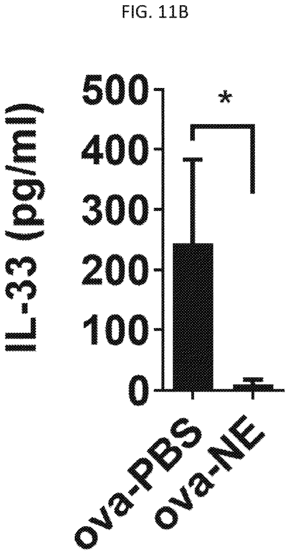

FIG. 11A is a graph showing IL-25 levels in lungs of mice following ova challenge. FIG. 11B is a graph showing IL-33 levels in lungs of mice following ova challenge. Data are expressed as mean ± standard deviation (n=8). Statistically significant differences (p<0.05) are indicated by *.

Figures 8a to 10c and 11a and 11b are for illustrative purposes, but not being part of the invention.

### DETAILED DESCRIPTION

The present disclosure is predicated, at least in part, on the discovery that a nanoemulsion-based vaccine improves airway function, reduced mucus production and airway pathology, and produces a significant decrease in the Th2 response in an animal model of chronic allergen-induced airway inflammation.

### Definitions

To facilitate an understanding of the present technology, a number of terms and phrases are defined below. Additional definitions are set forth throughout the detailed description.

The terms "disease" and "pathologic condition" are used interchangeably herein to describe a deviation from the condition regarded as normal or average for members of a species or group (e.g., humans), and which is detrimental to an affected individual under conditions that are not inimical to the majority of individuals of that species or group. Such a deviation can manifest as a state, signs, and/or symptoms (e.g., diarrhea, nausea, fever, pain, blisters, boils, rash, hyper-immune responses, hyper-sensitivity, immune suppression, inflammation, etc.) that are associated with any impairment of the normal state of a subject or of any of its organs or tissues that interrupts or modifies the performance of normal functions. A disease or pathological condition may be caused by or result from contact with a microorganism (e.g., a pathogen or other infective agent (e.g., a virus or bacteria)), may be responsive to environmental factors (e.g., allergens, malnutrition, industrial hazards, and/or climate), may be responsive to an inherent defect of the organism (e.g., genetic anomalies) or to combinations of these and other factors.

The term "allergy," as used herein, refers to a chronic condition involving an abnormal or pathological immune reaction to a substance (i.e., an "allergen") that is ordinarily harmless in average/healthy individuals. An "allergen" refers to any substance (e.g., an antigen) that induces an allergic reaction in a subject. Examples of allergens include, but are not limited to, aeroallergens (e.g., dust mite, mold, spores, plant pollens such as tree, weed, and grass pollens), food products (milk, egg, soy, wheat, nut, or fish proteins), animal products (e.g., cat or dog hair), drugs (e.g., penicillin), insect venom, and latex.

The terms "host" or "subject," as used herein, refer to an individual to be treated by (e.g., administered) the compositions and methods of the present disclosure. Subjects include, but are not limited to, mammals (e.g., murines, simians, equines, bovines, porcines, canines, felines, and the like), and preferably humans. In the context of the present disclosure, the term "subject" generally refers to an individual who will be administered or who has been administered one or more compositions described herein (e.g., a composition for inducing an immune response).

The term "emulsion," as used herein, includes classic oil-in-water or water-in-oil dispersions or droplets, as well as other lipid structures that can form as a result of hydrophobic forces that drive apolar residues (e.g., long hydrocarbon chains) away from water and drive polar head groups toward water, when a water immiscible oily phase is mixed with an aqueous phase. These other lipid structures include, but are not limited to, unilamellar, paucilamellar, and multilamellar lipid vesicles, micelles, and lamellar phases. Similarly, the term "nanoemulsion," as used herein, refers to oil-in-water dispersions comprising small lipid structures. For example, in some embodiments, nanoemulsions may comprise an oil phase having droplets with a mean particle size of approximately 0.1 to 5 microns (e.g., about 150, 200, 250, 300, 350, 400, 450, 500 nm or larger in diameter), although smaller and larger particle sizes are contemplated. The terms "emulsion" and "nanoemulsion" may be used interchangeably herein to refer to the nanoemulsions of the present disclosure.

The terms "surface active agent" and "surfactant," are used interchangeably herein and refer to amphipathic molecules that consist of a non-polar hydrophobic portion, usually a straight or branched hydrocarbon or fluorocarbon chain containing 8-18 carbon atoms, attached to a polar or ionic hydrophilic portion. The hydrophilic portion can be nonionic, ionic or zwitterionic. The hydrocarbon chain interacts weakly with the water molecules in an aqueous environment, whereas the polar or ionic head group interacts strongly with water molecules via dipole or ion-dipole interactions. Based on the nature of the hydrophilic group, surfactants are classified into anionic, cationic, zwitterionic, nonionic, and polymeric surfactants.

A used herein, the term "immune response" refers to a response by the immune system of a subject. Immune responses include, but are not limited to, a detectable alteration (e.g., increase) in Toll-like receptor (TLR) activation, lymphokine (e.g., cytokine (e.g., Th1 or Th2 type cytokines) or chemokine) expression and/or secretion, macrophage activation, dendritic cell activation, T cell activation (e.g., CD4+ or CD8+ T cells), natural killer (NK) cell activation, and/or B cell activation (e.g., antibody generation and/or secretion). Additional examples of immune responses include binding of an immunogen (e.g., antigen (e.g., immunogenic polypeptide)) to a major histocompatibility complex (MHC) molecule and inducing a cytotoxic T lymphocyte ("CTL") response, inducing a B cell response (e.g., antibody production), T-helper lymphocyte response, and/or a delayed type hypersensitivity (DTH) response against the antigen from which the immunogenic polypeptide is derived, expansion (e.g., growth of a population of cells) of cells of the immune system (e.g., T cells, B cells (e.g., of any stage of development (e.g., plasma cells)), and increased processing and presentation of antigen by antigen presenting cells. An immune response may be directed against immunogens that the subject's immune system recognizes as foreign (e.g., non-self antigens from microorganisms (e.g., pathogens), or self-antigens recognized as foreign). Thus, it is to be understood that, as used herein, "immune response" refers to any type of immune response, including, but not limited to, innate immune responses (e.g., activation of Toll receptor signaling cascade), cell-mediated immune responses (e.g., responses mediated by T cells (e.g., antigen-specific T cells) and non-specific cells of the immune system), and humoral immune responses (e.g., responses mediated by B cells (e.g., via generation and secretion of antibodies into the plasma, lymph, and/or tissue fluids)). The term "immune response" is meant to encompass all aspects of the capability of a subject's immune system to respond to antigens and/or immunogens (e.g., both the initial response to an immunogen (e.g., a pathogen) as well as acquired (e.g., memory) responses that are a result of an adaptive immune response).

As used herein, the term "immunity" refers to protection from disease (e.g., preventing or attenuating (e.g., suppression) of a sign, symptom, or condition of the disease) upon exposure to a substance or organism (e.g., antigen, allergen, or pathogen) capable of causing the disease. Immunity can be innate (e.g., non-adaptive (e.g., non-acquired) immune responses that exist in the absence of a previous exposure to an antigen) and/or acquired/adaptive (e.g., immune responses that are mediated by Band T cells following a previous exposure to antigen (e.g., that exhibit increased specificity and reactivity to the antigen)).

As used herein, the terms "immunogen" and "antigen" refer to an agent (e.g., a protein, an allergen, or a microorganism and/or portion or component thereof (e.g., a protein antigen (e.g., gp120 or rPA))) that is capable of eliciting an immune response in a subject. In preferred embodiments, immunogens elicit immunity against the immunogen (e.g., allergen) when administered in combination with a nanoemulsion of the present invention.

### Allergy and Aeroallergens

Allergic diseases, as well as inflammatory diseases, are associated with aberrant immune responses. For example, epithelial cells play an important role in orchestrating the allergic response, such as airway inflammation, through the release of multiple cytokines, including stem cell factor and several chemokines that attract eosinophils. T helper type 2 (Th2) cells orchestrate the inflammatory response in asthma, for example, through the release of IL-4 and IL-13 (which stimulate B cells to synthesize IgE), IL-5 (which is involved in eosinophilic inflammation), and IL-9 (which stimulates mast cell proliferation). Th2 cells predominate in most patients with allergies and asthma and differentiate from uncommitted precursor T cells under the influence of IL-4. T helper type 1 (Th1) cells differentiate under the influence of IL-12 and IL-27 and suppress Th2 cells through the release of IFN-γ. Th17 cells differentiate under the influence of IL-6 and IL-23. Regulatory T cells (Tregs) typically suppress other Th cells through the release of TGF-β and IL-10, and may have impaired function in allergic asthma.

IL-4 plays a critical role in the differentiation of Th2 cells from uncommitted Th0 cells and may be important in initial sensitization to allergens. It is also important for isotype switching of B cells from producers of IgG to producers of IgE. IL-13 mimics IL-4 in inducing IgE secretion and causing structural changes in the airways but does not play a role in promoting Th2 cell differentiation. IL-13 has attracted particular attention as a therapeutic target for the treatment of asthma, as it not only induces airway hyperresponsiveness (AHR) in animal models of asthma but also produces several of the structural changes seen in chronic asthma, including goblet cell hyperplasia, airway smooth muscle proliferation, and subepithelial fibrosis. IL-13 induces inflammation through stimulating the expression of multiple chemokines, including CCL11 (also known as eotaxin) from structural cells in the airways, including epithelial cells.

IL-9 overexpression in mice induces inflammation mediated by eosinophils, mucus hyperplasia, mastocytosis, AHR, and increased expression of other Th2 cytokines and IgE. IL-9 blockade inhibits pulmonary eosinophilia, mucus hypersecretion, and AHR after allergen challenge of sensitized mice. Asthmatic patients show increased expression of IL-9 and its receptor in the airways.

Accordingly, the present disclosure provide methods and compositions for the stimulation of immune responses and for treating or preventing allergic disease, particularly diseases associated with aeroallergen induced inflammation (e.g., airway inflammation). Specifically, the disclosure provides a composition comprising a nanoemulsion and one or more aeroallergens. The terms "aeroallergen" and "inhalant allergen" are used interchangeably herein and refer to an allergen that is airborne. Through sensitization and other innate mechanisms, aeroallergens have been associated with mucosal inflammation in pathologies ranging from reactive airway disease to allergic rhinitis. Not to be bound by any particular theory, it is believed that aeroallergens, regardless of the extent of penetration into sinuses, cause a systemic allergic response (London et al., World J Otorhinolaryngol Head Neck Surg., 4(3): 209-215 (2018)).

The composition of the invention comprises cockroach allergen. Other aeroallergens known in the art, for illustrative purposes, but not being part of the invention, include, for example, mold spores, dust mites, animal hair, animal urine, dust, cosmetics (e.g., perfumes), plant pollens (e.g., tress, grass, and/or weed pollens), weeds, grass, air pollution, and any component thereof (see, e.g., Adkinson et al. (eds)., Middleton 's Allergy: Principles and Practice, 8th Edition, Elsevier (2013)). It will be appreciated that a whole allergen may contain more than one allergenic proteins. Thus, the compositions described herein comprise any allergenic component of a cockroach allergen source.

### Nanoemulsions

The nanoemulsion of the invention is mixed with a cockroach allergen that causes an allergic response (e.g., aeroallergen induced inflammation (e.g., airway inflammation)) to generate the composition of the present invention. The nanoemulsion comprises (a) a poloxamer surfactant or polysorbate surfactant; (b) an organic solvent; (c) a halogen-containing compound; (d) oil, and (e) water. In this regard, the nanoemulsion comprises an aqueous phase, such as, for example, water (e.g., distilled water, purified water, water for injection, de-ionized water, tap water, etc.) and solutions (e.g., phosphate buffered saline (PBS) solution). In certain embodiments, the aqueous phase comprises water at a pH of about 4 to 10, preferably about 6 to 8. The water can be deionized (hereinafter "DiHzO"). In some embodiments, the aqueous phase comprises phosphate buffered saline (PBS). The aqueous phase may further be sterile and pyrogen free.

The nanoemulsion comprises a suitable organic solvent. Suitable organic solvents include, but are not limited to, C₁-C₁₂ alcohol, diol, triol, dialkyl phosphate, tri-alkyl phosphate (e.g., tri-n-butyl phosphate), semi-synthetic derivatives thereof, and combinations thereof. In one aspect, the organic solvent is an alcohol chosen from a nonpolar solvent, a polar solvent, a protic solvent, or an aprotic solvent. Other suitable organic solvents for the nanoemulsion include, but are not limited to, ethanol, methanol, isopropyl alcohol, propanol, octanol, glycerol, medium chain triglycerides, diethyl ether, ethyl acetate, acetone, dimethyl sulfoxide (DMSO), acetic acid, n-butanol, butylene glycol, perfumers alcohols, isopropanol, n-propanol, formic acid, propylene glycols, sorbitol, industrial methylated spirit, triacetin, hexane, benzene, toluene, diethyl ether, chloroform, 1,4-dixoane, tetrahydrofuran, dichloromethane, acetone, acetonitrile, dimethylformamide, dimethyl sulfoxide, formic acid, polyethylene glycol, an organic phosphate based solvent, semi-synthetic derivatives thereof, and any combination thereof.

The oil phase is any cosmetically or pharmaceutically acceptable oil. The oil can be volatile or non-volatile, and may be chosen from animal oil, vegetable oil, natural oil, synthetic oil, hydrocarbon oils, silicone oils, semi-synthetic derivatives thereof, and combinations thereof. Examples of suitable oils that may be used in the nanoemulsion include mineral oil, squalene oil, flavor oils, silicon oil, essential oils, water insoluble vitamins, isopropyl stearate, butyl stearate, octyl palmitate, cetyl palmitate, tridecyl behenate, diisopropyl adipate, dioctyl sebacate, menthyl anthranhilate, cetyl octanoate, octyl salicylate, isopropyl myristate, neopentyl glycol dicarpate cetols, ceraphyls, decyl oleate, diisopropyl adipate, C₁₂₋₁₅ alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, canola oil, chile oil, coconut oil, corn oil, cottonseed oil, flaxseed oil, grape seed oil, mustard oil, olive oil, palm oil, palm kernel oil, peanut oil, pine seed oil, poppy seed oil, pumpkin seed oil, rice bran oil, safflower oil, tea oil, truffle oil, vegetable oil, apricot (kernel) oil, jojoba oil (*Simmondsia chinensis* seed oil), macadamia oil, wheat germ oil, almond oil, rapeseed oil, gourd oil, soybean oil, sesame oil, hazelnut oil, maize oil, sunflower oil, hemp oil, bois oil, kuki nut oil, avocado oil, walnut oil, fish oil, berry oil, allspice oil, juniper oil, seed oil, almond seed oil, anise seed oil, celery seed oil, cumin seed oil, nutmeg seed oil, leaf oil, basil leaf oil, bay leaf oil, cinnamon leaf oil, common sage leaf oil, eucalyptus leaf oil, lemon grass leaf oil, melaleuca leaf oil, oregano leaf oil, patchouli leaf oil, peppermint leaf oil, pine needle oil, rosemary leaf oil, spearmint leaf oil, tea tree leaf oil, thyme leaf oil, wintergreen leaf oil, flower oil, chamomile oil, clary sage oil, clove oil, geranium flower oil, hyssop flower oil, jasmine flower oil, lavender flower oil, manuka flower oil, marhoram flower oil, orange flower oil, rose flower oil, ylang-ylang flower oil, vark oil, cassia bark oil, cinnamon bark oil, sassafras bark oil, wood oil, camphor wood oil, cedar wood oil, rosewood oil, sandalwood oil, rhizome (ginger) wood oil, resin oil, frankincense oil, myrrh oil, peel oil, bergamot peel oil, grapefruit peel oil, lemon peel oil, lime peel oil, orange peel oil, tangerine peel oil, root oil, valerian oil, oleic acid, linoleic acid, oleyl alcohol, isostearyl alcohol, semi-synthetic derivatives thereof, and any combinations thereof.

The oil may further comprise a silicone component, such as a volatile silicone component, which can be the sole oil in the silicone component or can be combined with other silicone and non-silicone, volatile and non-volatile oils. Suitable silicone components include, but are not limited to, methylphenylpolysiloxane, simethicone, dimethicone, phenyltrimethicone (or an organo-modified version thereof), alkylated derivatives of polymeric silicones, cetyl dimethicone, lauryl trimethicone, hydroxylated derivatives of polymeric silicones, such as dimethiconol, volatile silicone oils, cyclic and linear silicones, cyclomethicone, derivatives of cyclomethicone, hexamethylcyclotrisiloxane, octamethylcyclotetrasiloxane, decamethylcyclopentasiloxane, volatile linear dimethylpolysiloxanes, isohexadecane, isoeicosane, isotetracosane, polyisobutene, isooctane, isododecane, semi-synthetic derivatives thereof, and combinations thereof.

The volatile oil can be the organic solvent, or the volatile oil can be present in addition to an organic solvent. Suitable volatile oils include, but are not limited to, a terpene, monoterpene, sesquiterpene, carminative, azulene, menthol, camphor, thujone, thymol, nerol, linalool, limonene, geraniol, perillyl alcohol, nerolidol, farnesol, ylangene, bisabolol, farnesene, ascaridole, chenopodium oil, citronellal, citral, citronellol, chamazulene, yarrow, guaiazulene, chamomile, semi-synthetic derivatives thereof, or combinations thereof. In certain embodiments, the volatile oil in the silicone component is different than the oil in the oil phase.

The surfactant in the nanoemulsion is a poloxamer or a polysorbate. Other surfactants, for illustrative purposes, not being part of the invention, may be a pharmaceutically acceptable ionic surfactant, a pharmaceutically acceptable nonionic surfactant, a pharmaceutically acceptable cationic surfactant, a pharmaceutically acceptable anionic surfactant, or a pharmaceutically acceptable zwitterionic surfactant. Exemplary useful surfactants are described in, e.g., Applied Surfactants: Principles and Applications, Tharwat F. Tadros, WILEY-VCH Verlag GmbH & Co. KGaA, Weinheim (2005)). In other embodiments, the surfactant may be a pharmaceutically acceptable ionic polymeric surfactant, a pharmaceutically acceptable nonionic polymeric surfactant, a pharmaceutically acceptable cationic polymeric surfactant, a pharmaceutically acceptable anionic polymeric surfactant, or a pharmaceutically acceptable zwitterionic polymeric surfactant. Examples of polymeric surfactants include, but are not limited to, a graft copolymer of a poly(methyl methacrylate) backbone with multiple (at least one) polyethylene oxide (PEO) side chain, polyhydroxystearic acid, an alkoxylated alkyl phenol formaldehyde condensate, a polyalkylene glycol modified polyester with fatty acid hydrophobes, a polyester, semi-synthetic derivatives thereof, or combinations thereof.

Specific examples of suitable surfactants include ethoxylated nonylphenol comprising 9 to 10 units of ethyleneglycol, ethoxylated undecanol comprising 8 units of ethyleneglycol, polyoxyethylene (20) sorbitan monolaurate, polyoxyethylene (20) sorbitan monopalmitate, polyoxyethylene (20) sorbitan monostearate, polyoxyethylene (20) sorbitan monooleate, sorbitan monolaurate, sorbitan monopalmitate, sorbitan monostearate, sorbitan monooleate, ethoxylated hydrogenated ricin oils, sodium laurylsulfate, a diblock copolymer of ethyleneoxyde and propyleneoxyde, ethylene oxide-propylene oxide block copolymers, and tetra-functional block copolymers based on ethylene oxide and propylene oxide, glyceryl monoesters, glyceryl caprate, glyceryl caprylate, glyceryl cocate, glyceryl erucate, glyceryl hydroxysterate, glyceryl isostearate, glyceryl lanolate, glyceryl laurate, glyceryl linolate, glyceryl myristate, glyceryl oleate, glyceryl PABA, glyceryl palmitate, glyceryl ricinoleate, glyceryl stearate, glyceryl thighlycolate, glyceryl dilaurate, glyceryl dioleate, glyceryl dimyristate, glyceryl disterate, glyceryl sesuioleate, glyceryl stearate lactate, polyoxyethylene cetyl/stearyl ether, polyoxyethylene cholesterol ether, polyoxyethylene laurate or dilaurate, polyoxyethylene stearate or distearate, polyoxyethylene fatty ethers, polyoxyethylene lauryl ether, polyoxyethylene stearyl ether, polyoxyethylene myristyl ether, a steroid, cholesterol, betasitosterol, bisabolol, fatty acid esters of alcohols, isopropyl myristate, aliphati-isopropyl n-butyrate, isopropyl n-hexanoate, isopropyl n-decanoate, isoproppyl palmitate, octyldodecyl myristate, alkoxylated alcohols, alkoxylated acids, alkoxylated amides, alkoxylated sugar derivatives, alkoxylated derivatives of natural oils and waxes, polyoxyethylene polyoxypropylene block copolymers, nonoxynol-14, PEG-8 laurate, PEG-6 cocoamide, PEG-20 methylglucose sesquistearate, PEG40 lanolin, PEG-40 castor oil, PEG-40 hydrogenated castor oil, polyoxyethylene fatty ethers, glyceryl diesters, polyoxyethylene stearyl ether, polyoxyethylene myristyl ether, and polyoxyethylene lauryl ether, glyceryl dilaurate, glyceryl dimystate, glyceryl distearate, semi-synthetic derivatives thereof, or mixtures thereof.

Additional suitable surfactants include, but are not limited to, non-ionic lipids, such as glyceryl laurate, glyceryl myristate, glyceryl dilaurate, glyceryl dimyristate, semi-synthetic derivatives thereof, and mixtures thereof.

In other embodiments, the surfactant may be a polyoxyethylene fatty ether having a polyoxyethylene head group ranging from about 2 to about 100 groups, or an alkoxylated alcohol having the structure R₅-(OCH₂CH₂)y-OH, wherein Rs is a branched or unbranched alkyl group having from about 6 to about 22 carbon atoms and y is between about 4 and about 100, preferably between about 10 and about 100. Preferably, the alkoxylated alcohol is the species wherein Rs is a lauryl group and y has an average value of 23.

In other embodiments, the surfactant may be an alkoxylated alcohol which is an ethoxylated derivative of lanolin alcohol. For example, the ethoxylated derivative of lanolin alcohol may be laneth-10, which is the polyethylene glycol ether of lanolin alcohol with an average ethoxylation value of 10.

Nonionic surfactants include, but are not limited to, an ethoxylated surfactant, an alcohol ethoxylated, an alkyl phenol ethoxylated, a fatty acid ethoxylated, a monoalkaolamide ethoxylated, a sorbitan ester ethoxylated, a fatty amino ethoxylated, an ethylene oxide-propylene oxide copolymer, Bis(polyethylene glycol bis(imidazoyl carbonyl)), nonoxynol-9, Bis(polyethylene glycol bis[imidazoyl carbonyl]), BRIJ 35, BRIJ 56, BRIJ 72, BRIJ 76, BRIJ 92V, BRIJ 97, BRIJ 58P, CREMOPHOR, EL, decaethylene glycol monododecyl ether, N-decanoyl-N-methylglucamine, n-decyl alpha-D-glucopyranoside, decyl beta-D-maltopyranoside, n-dodecanoyl-N-methylglucamide, n-dodecyl alpha-D-maltoside, n-dodecyl beta-D-maltoside, n-dodecyl beta-D-maltoside, heptaethylene glycol monodecyl ether, heptaethylene glycol monododecyl ether, heptaethylene glycol monotetradecyl ether, n-hexadecyl beta-D-maltoside, hexaethylene glycol monododecyl ether, hexaethylene glycol monohexadecyl ether, hexaethylene glycol monooctadecyl ether, hexaethylene glycol monotetradecyl ether, igepal CA-630, igepal CA-630, Methyl-6-O-(N-heptylcarbamoyl)-alpha-D-glucopyranoside, nonaethylene glycol monododecyl ether, N-nonanoyl-N-methylglucamine, N-nonanoyl-N-methylglucamine, octaethylene glycol monodecyl ether, octaethylene glycol monododecyl ether, octaethylene glycol monohexadecyl ether, octaethylene glycol monooctadecyl ether, octaethylene glycol monotetradecyl ether, octyl-beta-D-glucopyranoside, pentaethylene glycol monodecyl ether, pentaethylene glycol monododecyl ether, pentaethylene glycol monohexadecyl ether, pentaethylene glycol monohexyl ether, pentaethylene glycol monooctadecyl ether, pentaethylene glycol monooctyl ether, polyethylene glycol diglycidyl ether, polyethylene glycol ether W-1, polyoxyethylene 10 tridecyl ether, polyoxyethylene 100 stearate, polyoxyethylene 20 isohexadecyl ether, polyoxyethylene 20 oleyl ether, polyoxyethylene 40 stearate, polyoxyethylene 50 stearate, polyoxyethylene 8 stearate, polyoxyethylene bis(imidazolyl carbonyl), polyoxyethylene 25 propylene glycol stearate, Saponin from Quillaja bark, SPAN 20, SPAN 40, SPAN 60, SPAN 65, SPAN 80, SPAN 85, tergitol, type 15-S-12, tergitol, type 15-S-30, tergitol, type 15-S-5, tergitol, type 15-S-7, tergitol, Type 15-S-9, tergitol, type NP-10, tergitol, type NP-4, tergitol, type NP-40, tergitol, type NP-7, tergitol, type NP-9, tergitol, tergitol, type TMN-10, tergitol, type TMN-6, tetradecyl-beta-D-maltoside, tetraethylene glycol monodecyl ether, tetraethylene glycol monododecyl ether, tetraethylene glycol monotetradecyl ether, triethylene glycol monodecyl ether, triethylene glycol monododecyl ether, triethylene glycol monohexadecyl ether, triethylene glycol monooctyl ether, triethylene glycol monotetradecyl ether, triton CF-21, triton CF-32, triton DF-12, triton DF-16, triton GR-5M, triton QS-15, triton QS-44, triton X-100, triton X-102, triton X-15, triton X-151, triton X-200, triton X-207, TRITON X-100, TRITON X-114, TRITON X-165, TRITON X-305, TRITON X-405, TRITON X-45, TRITON X-705-70, TWEEN 20, TWEEN 21, TWEEN 40, TWEEN 60, TWEEN 61, TWEEN 65, TWEEN 80, TWEEN 81, TWEEN 85, tyloxapol, n-undecyl beta-D-glucopyranoside, semi-synthetic derivatives thereof, or combinations thereof.

In a specific embodiment, the nonionic surfactant may be a poloxamer. Poloxamers are polymers made of a block of polyoxyethylene, followed by a block of polyoxypropylene, followed by a block of polyoxyethylene. The average number of units of polyoxyethylene and polyoxypropylene varies based on the number associated with the polymer. For example, the smallest polymer, poloxamer 101, consists of a block with an average of 2 units of polyoxyethylene, a block with an average of 16 units of polyoxypropylene, followed by a block with an average of 2 units of polyoxyethylene. Poloxamers range from colorless liquids and pastes to white solids. In cosmetics and personal care products, poloxamers are used in the formulation of skin cleansers, bath products, shampoos, hair conditioners, mouthwashes, eye makeup remover, and other skin and hair products. Examples of poloxamers include, but are not limited to, poloxamer 101, poloxamer 105, poloxamer 108, poloxamer 122, poloxamer 123, poloxamer 124, poloxamer 181, poloxamer 182, poloxamer 183, poloxamer 184, poloxamer 185, poloxamer 188, poloxamer 212, poloxamer 215, poloxamer 217, poloxamer 231, poloxamer 234, poloxamer 235, poloxamer 237, poloxamer 238, poloxamer 282, poloxamer 284, poloxamer 288, poloxamer 331, poloxamer 333, poloxamer 334, poloxamer 335, poloxamer 338, poloxamer 401, poloxamer 402, poloxamer 403, poloxamer 407, poloxamer 105 benzoate, and poloxamer 182 dibenzoate.

In another embodiment, the nonionic surfactant may be a polysorbate surfactant, such as polysorbate 20 or polysorbate 80. For example, polysorbate 80 may be included in the nanoemulsion at a concentration of about 0.01% to about 5.0% (e.g., about 0.05%, about 0.08%, about 0.1%, about 0.5%, about 1.0%, about 1.5%, about 2.0%, about 2.5%, about 3.0%, about 3.5%, about 4.0%, or about 4.5%). In one embodiment, polysorbate 80 is included in the nanoemulsion at a concentration of about 0.1% to about 3% (e.g., about 0.3%, about 0.4%, about 0.6%, about 0.9%, about 1.2%, about 1.4%, about 1.6%, about 1.8%, about 2.1%, about 2.3%, about 2.5%, about 2.7%, or about 2.9%).

Suitable cationic surfactants include, but are not limited to, a quarternary ammonium compound, an alkyl trimethyl ammonium chloride compound, a dialkyl dimethyl ammonium chloride compound, a cationic halogen-containing compound, such as cetylpyridinium chloride, benzalkonium chloride, benzalkonium chloride, benzyldimethylhexadecylammonium chloride, benzyldimethyltetradecylammonium chloride, benzyldodecyldimethylammonium bromide, benzyltrimethylammonium tetrachloroiodate, dimethyldioctadecylammonium bromide, dodecylethyldimethylammonium bromide, dodecyltrimethylammonium bromide, dodecyltrimethylammonium bromide, ethylhexadecyldimethylammonium bromide, Girard's reagent T, hexadecyltrimethylammonium bromide, hexadecyltrimethylammonium bromide, N,N',N'-polyoxyethylene(10)-N-tallow-1,3-diaminopropane, thonzonium bromide, trimethyl(tetradecyl)ammonium bromide, 1,3,5-Triazine-1,3,5(2H,4H,6H)-triethanol, 1-decanaminium, N-decyl-N,N-dimethyl-, chloride, didecyl dimethyl ammonium chloride, 2-(2-(p-(diisobutyl)cresosxy)ethoxy)ethyl dimethyl benzyl ammonium chloride, 2-(2-(p-(diisobutyl)phenoxy)ethoxy)ethyl dimethyl benzyl ammonium chloride, alkyl 1 or 3 benzyl-1-(2-hydroxethyl)-2-imidazolinium chloride, alkyl bis(2-hydroxyethyl) benzyl ammonium chloride, alkyl demethyl benzyl ammonium chloride, alkyl dimethyl 3,4-dichlorobenzyl ammonium chloride (100% 012), alkyl dimethyl 3,4-dichlorobenzyl ammonium chloride (50% 014, 40% C12, 10% 016), alkyl dimethyl 3,4-dichlorobenzyl ammonium chloride (55% C14, 23% C12, 20% 016), alkyl dimethyl benzyl ammonium chloride, alkyl dimethyl benzyl ammonium chloride (100% 014), alkyl dimethyl benzyl ammonium chloride (100% C16), Alkyl dimethyl benzyl ammonium chloride (41% C14, 28% C12), alkyl dimethyl benzyl ammonium chloride (47% C12, 18% C14), alkyl dimethyl benzyl ammonium chloride (55% C16, 20% C14), alkyl dimethyl benzyl ammonium chloride (58% C14, 28% C16), alkyl dimethyl benzyl ammonium chloride (60% C14, 25% C12), alkyl dimethyl benzyl ammonium chloride (61% C11, 23% C14), alkyl dimethyl benzyl ammonium chloride (61% C12, 23% C14), alkyl dimethyl benzyl ammonium chloride (65% C12, 25% C14), alkyl dimethyl benzyl ammonium chloride (67% C12, 24% C14), alkyl dimethyl benzyl ammonium chloride (67% C12, 25% C14), alkyl dimethyl benzyl ammonium chloride (90% C14, 5% C12), alkyl dimethyl benzyl ammonium chloride (93% C14, 4% C12), alkyl dimethyl benzyl ammonium chloride (95% C16, 5% C18), alkyl dimethyl benzyl ammonium chloride, alkyl didecyl dimethyl ammonium chloride, alkyl dimethyl benzyl ammonium chloride, alkyl dimethyl benzyl ammonium chloride (C12-16), alkyl dimethyl benzyl ammonium chloride (C12-18), alkyl dimethyl benzyl ammonium chloride, dialkyl dimethyl benzyl ammonium chloride, alkyl dimethyl dimethybenzyl ammonium chloride, alkyl dimethyl ethyl ammonium bromide (90% C14, 5% C16, 5% C12), alkyl dimethyl ethyl ammonium bromide (mixed alkyl and alkenyl groups as in the fatty acids of soybean oil), alkyl dimethyl ethylbenzyl ammonium chloride, alkyl dimethyl ethylbenzyl ammonium chloride (60% C14), alkyl dimethyl isopropylbenzyl ammonium chloride (50% C12, 30% C14, 17% C16, 3% C18), alkyl trimethyl ammonium chloride (58% C18, 40% C16, 1% C14, 1% C12), Alkyl trimethyl ammonium chloride (90% C18, 10% C16), alkyldimethyl-(ethylbenzyl) ammonium chloride (C12-18), di-(C8-10)-alkyl dimethyl ammonium chlorides, dialkyl dimethyl ammonium chloride, dialkyl methyl benzyl ammonium chloride, didecyl dimethyl ammonium chloride, diisodecyl dimethyl ammonium chloride, dioctyl dimethyl ammonium chloride, dodecyl bis(2-hydroxyethyl) octyl hydrogen ammonium chloride, dodecyl dimethyl benzyl ammonium chloride, dodecylcarbamoyl methyl dimethyl benzyl ammonium chloride, heptadecyl hydroxyethylimidazolinium chloride, hexahydro-1,3,5-tris(2-hydroxyethyl)-s-triazine, hexahydro-1,3,5-tris(2-hydroxyethyl)-s-triazine, myristalkonium chloride (and) Quat RNIUM 14, N,N-dimethyl-2-hydroxypropylammonium chloride polymer, n-Tetradecyl dimethyl benzyl ammonium chloride monohydrate, octyl decyl dimethyl ammonium chloride, octyl dodecyl dimethyl ammonium chloride, octyphenoxyethoxyethyl dimethyl benzyl ammonium chloride, oxydiethylenebis(alkyl dimethyl ammonium chloride), quaternary ammonium compounds, dicoco alkyldimethyl, chloride, trimethoxysily propyl dimethyl octadecyl ammonium chloride, trimethoxysilyl quats, trimethyl dodecylbenzyl ammonium chloride, semi-synthetic derivatives thereof, and combinations thereof.

Exemplary cationic halogen-containing compounds include, but are not limited to, cetylpyridinium halides, cetyltrimethylammonium halides, cetyldimethylethylammonium halides, cetyldimethylbenzylammonium halides, cetyltributylphosphonium halides, dodecyltrimethylammonium halides, or tetradecyltrimethylammonium halides. In some embodiments, cationic halogen-containing compounds include, but are not limited to, cetylpyridinium chloride (CPC), cetyltrimethylammonium chloride, cetylbenzyldimethylammonium chloride, cetylpyridinium bromide (CPB), cetyltrimethylammonium bromide (CTAB), cetyidimethylethylammonium bromide, cetyltributylphosphonium bromide, dodecyltrimethylammonium bromide, and tetrad ecyltrimethylammonium bromide. In particularly preferred embodiments, the cationic halogen-containing compound is CPC, although the compositions of the present invention are not limited to formulation with an particular cationic containing compound.

Suitable anionic surfactants include, but are not limited to, a carboxylate, a sulphate, a sulphonate, a phosphate, chenodeoxycholic acid, chenodeoxycholic acid sodium salt, cholic acid, ox or sheep bile, dehydrocholic acid, deoxycholic acid, deoxycholic acid, deoxycholic acid methyl ester, digitonin, digitoxigenin, N,N-dimethyldodecylamine N-oxide, docusate sodium salt, glycochenodeoxycholic acid sodium salt, glycocholic acid hydrate, synthetic, glycocholic acid sodium salt hydrate, synthetic, glycodeoxycholic acid monohydrate, Glycodeoxycholic acid sodium salt, glycodeoxycholic acid sodium salt, glycolithocholic acid 3-sulfate disodium salt, glycolithocholic acid ethyl ester, N-lauroylsarcosine sodium salt, N-lauroylsarcosine solution, N-lauroylsarcosine solution, lithium dodecyl sulfate, lithium dodecyl sulfate, lithium dodecyl sulfate, lugol solution, Niaproof 4, yype 4, 1-Octanesulfonic acid sodium salt, sodium 1-butanesulfonate, sodium 1-decanesulfonate, sodium 1-decanesulfonate, sodium 1-dodecanesulfonate, sodium 1-heptanesulfonate anhydrous, sodium 1-heptanesulfonate anhydrous, sodium 1-nonanesulfonate, sodium 1-propanesulfonate monohydrate, sodium 2-bromoethanesulfonate, sodium cholate hydrate, sodium choleate, sodium deoxycholate, sodium deoxycholate monohydrate, sodium dodecyl sulfate, sodium hexanesulfonate anhydrous, sodium octyl sulfate, sodium pentanesulfonate anhydrous, sodium taurocholate, taurochenodeoxycholic acid sodium salt, taurodeoxycholic acid sodium salt monohydrate, taurohyodeoxycholic acid sodium salt hydrate, taurolithocholic acid 3-sulfate disodium salt, tauroursodeoxycholic acid sodium salt, TRIZMA dodecyl sulfate, TWEEN 80, ursodeoxycholic acid, semi-synthetic derivatives thereof, and combinations thereof.

Suitable zwitterionic surfactants include, but are not limited to, an N-alkyl betaine, lauryl amindo propyl dimethyl betaine, an alkyl dimethyl glycinate, an N-alkyl amino propionate, CHAPS, minimum 98% (TLC), CHAPS, SigmaUltra, minimum 98% (TLC), CHAPS, for electrophoresis, minimum 98% (TLC), CHAPSO, minimum 98%, CHAPSO, SigmaUltra, CHAPSO, for electrophoresis, 3 -(decyldimethylammonio)propanesulfonate inner salt, 3-dodecyldimethyl-ammonio)propanesulfonate inner salt, SigmaUltra, 3-(dodecyldimethylammonio)propanesulfonate inner salt, 3-(N,N-dimethylmyristylammonio)propanesulfonate, 3-(N,N-dimethylocatdecylammonio)propanesulfonate, 3-(N,N-dimethyloctyl-ammonio)propanesulfonate inner salt, 3-(N,N-dimethylpalmitylammonio)-propanesulfonate, semi-synthetic derivatives thereof, and combinations thereof.

In some embodiments, the nanoemulsion comprises a cationic surfactant, which can be cetylpyridinium chloride (CPC). When the nanoemulsion comprises a cationic surfactant, the concentration of the cationic surfactant desirably is less than about 5.0% and greater than about 0.001%. For example, the concentration of the cationic surfactant may be less than about 5%, less than about 4.5%, less than about 4.0%, less than about 3.5%, less than about 3.0%, less than about 2.5%, less than about 2.0%, less than about 1.5%, less than about 1.0%, less than about 0.90%, less than about 0.80%, less than about 0.70%, less than about 0.60%, less than about 0.50%, less than about 0.40%, less than about 0.30%, less than about 0.20%, or less than about 0.10%. The concentration of the cationic agent in the nanoemulsion desirably is greater than about 0.002%, greater than about 0.003%, greater than about 0.004%, greater than about 0.005%, greater than about 0.006%, greater than about 0.007%, greater than about 0.008%, greater than about 0.009%, greater than about 0.010%, or greater than about 0.001%.

Alternatively, the nanoemulsion may comprise at least one cationic surfactant and at least one non-cationic surfactant. The non-cationic surfactant may be a nonionic surfactant, such as a polysorbate (Tween) (e.g., polysorbate 80 or polysorbate 20). In one embodiment, the concentration of the non-ionic surfactant is about 0.01% to about 5.0%, e.g., about 0.1% to about 3%, and the concentration of the cationic surfactant is about 0.01% to about 2%.

In certain embodiments, the nanoemulsion further comprises a halogen-containing compound, such as a cationic halogen-containing compound. The present disclosure is not limited to a particular cationic halogen-containing compound. A variety of cationic halogen-containing compounds may be included in the nanoemulsion, such as, for example, cetylpyridinium halides, cetyltrimethylammonium halides, cetyldimethylethylammonium halides, cetyldimethylbenzylammonium halides, cetyltributylphosphonium halides, dodecyltrimethylammonium halides, and tetradecyltrimethylammonium halides. The disclosed nanoemulsion composition also is not limited to a particular halide. A variety of halides may be included in the nanoemulsion composition, such as, for example, chloride, fluoride, bromide, and iodide.

The nanoemulsion may further comprise a quaternary ammonium-containing compound. Suitable quaternary ammonium-containing compounds that may be incorporated in the nanoemulsion include, but are not limited to, alkyl dimethyl benzyl ammonium chloride, dialkyl dimethyl ammonium chloride, n-alkyl dimethyl benzyl ammonium chloride, n-alkyl dimethyl ethylbenzyl ammonium chloride, dialkyl dimethyl ammonium chloride, and n-alkyl dimethyl benzyl ammonium chloride.

The present composition may comprise compounds or components in addition those described above. Such additional compounds include, but are not limited to, one or more solvents, such as an organic phosphate-based solvent, bulking agents, coloring agents, pharmaceutically acceptable excipients, a preservative, pH adjuster, buffer, chelating agent, etc. The additional compounds can be admixed into a previously emulsified composition comprising a nanoemulsion, or the additional compounds can be added to the original mixture to be emulsified. In certain embodiments, one or more additional compounds are admixed into an existing immunogenic composition immediately prior to its use.

Suitable preservatives that can be employed in the composition include, but are not limited to, cetylpyridinium chloride, benzalkonium chloride, benzyl alcohol, chlorhexidine, imidazolidinyl urea, phenol, potassium sorbate, benzoic acid, bronopol, chlorocresol, sorbic acid, alpha-tocophernol, ascorbic acid, ascorbyl palmitate, butylated hydroxyanisole, butylated hydroxytoluene, sodium ascorbate, sodium metabisulphite, citric acid, edetic acid, chlorphenesin (3-(-4-chloropheoxy)-propane-1,2-diol), Kathon CG (methyl and methylchloroisothiazolinone), parabens (methyl, ethyl, propyl, butyl hydrobenzoates), phenoxyethanol (2-phenoxyethanol), sorbic acid (potassium sorbate, sorbic acid), Phenonip (phenoxyethanol, methyl, ethyl, butyl, propyl parabens), Phenoroc (phenoxyethanol 0.73%, methyl paraben 0.2%, propyl paraben 0.07%), Liquipar Oil (isopropyl, isobutyl, butylparabens), Liquipar PE (70% phenoxyethanol, 30% liquipar oil), Nipaguard MPA (benzyl alcohol (70%), methyl & propyl parabens), Nipaguard MPS (propylene glycol, methyl & propyl parabens), Nipasept (methyl, ethyl and propyl parabens), Nipastat (methyl, butyl, ethyl and propyel parabens), Elestab 388 (phenoxyethanol in propylene glycol plus chlorphenesin and methylparaben), Killitol (7.5% chlorphenesin and 7.5% methyl parabens), semi-synthetic derivatives thereof, and combinations thereof.

The disclosed composition may further comprise at least one pH adjuster, such as, for example, diethyanolamine, lactic acid, monoethanolamine, triethylanolamine, sodium hydroxide, sodium phosphate, semi-synthetic derivatives thereof, and combinations thereof.

The disclosed composition may further comprise a chelating agent. In one embodiment, the chelating agent may be present in an amount of about 0.0005% to about 1%. Examples of chelating agents include, but are not limited to, ethylenediamine, ethylenediaminetetraacetic acid (EDTA), phytic acid, polyphosphoric acid, citric acid, gluconic acid, acetic acid, lactic acid, and dimercaprol.

The composition may further comprise a buffering agent, such as a pharmaceutically acceptable buffering agent. Examples of buffering agents include, but are not limited to, 2-amino-2-methyl-1,3-propanediol, ?99.5% (NT), 2-amino-2-methyl-1-propanol, ?99.0% (GC), L-(+)-tartaric acid, ?99.5% (T), ACES, ?99.5% (T), ADA, ≧99.0% (T), acetic acid, ≧99.5% (GC/T), acetic acid, for luminescence, ≧99.5% (GC/T), ammonium acetate solution, for molecular biology, about 5 M in H₂O, ammonium acetate, for luminescence, ≧99.0% (calc. on dry substance, T), ammonium bicarbonate, ?99.5% (T), ammonium citrate dibasic, ≧99.00% (T), ammonium formate solution, 10 M in H₂O, ammonium formate, ≧99.0% (calc. based on dry substance, NT), ammonium oxalate monohydrate, ≧99.5% (RT), ammonium phosphate dibasic solution, 2.5 M in H₂O, ammonium phosphate dibasic, ?99.0% (T), ammonium phosphate monobasic solution, 2.5 M in H₂O, ammonium phosphate monobasic, ≧99.5% (T), ammonium sodium phosphate dibasic tetrahydrate, ≧99.5% (NT), ammonium sulfate solution, for molecular biology, 3.2 M in H₂O, ammonium tartrate dibasic solution, 2 M in H₂O (colorless solution at 20.degree. C.), ammonium tartrate dibasic, ≧99.5% (T), BES buffered saline, for molecular biology, 2.times. concentrate, BES, ?99.5% (T), BES, for molecular biology, ≧99.5% (T), BICINE buffer Solution, for molecular biology, 1 M in H₂O, BICINE, ≧99.5% (T), BIS-TR1S, ≧99.0% (NT), bicarbonate buffer solution, >0.1 M Na₂CO₃, >0.2 M NaHCO₃, boric acid, ≧99.5% (T), boric acid, for molecular biology, ≧99.5% (T), CAPS, ≧99.0% (TLC), CHES, ≧99.5% (T), calcium acetate hydrate, ≧99.0% (calc. on dried material, KT), calcium carbonate, precipitated, §99.0% (KT), calcium citrate tribasic tetrahydrate, ?98.0% (calc. on dry substance, KT), citrate concentrated solution, for molecular biology, 1 M in H₂O, citric acid, anhydrous, ≧99.5% (T), citric acid, for luminescence, anhydrous, ≧99.5% (T), diethanolamine, ≧99.5% (GC), EPPS, ≧99.0% (T), ethylenediaminetetraacetic acid disodium salt dihydrate, for molecular biology, ≧99.0% (T), formic acid solution, 1.0 M in H₂O, Gly-Gly-Gly, ≧99.0% (NT), Gly-Gly, ≧99.5% (NT), glycine, ≧99.0% (NT), glycine, for luminescence, ≧99.0% (NT), glycine, for molecular biology, ≧99.0% (NT), HEPES buffered saline, for molecular biology, 2.times. concentrate, HEPES, ≧99.5% (T), HEPES, for molecular biology, ?99.5% (T), imidazole buffer solution, 1 M in H₂O, imidazole, ≧99.5% (GC), imidazole, for luminescence, ≧99.5% (GC), imidazole, for molecular biology, ?99.5% (GC), lipoprotein refolding buffer, lithium acetate dihydrate, >99.0% (NT), lithium citrate tribasic tetrahydrate, ≧99.5% (NT), MES hydrate, ≧99.5% (T), MES monohydrate, for luminescence, ≧99.5% (T), MES solution, for molecular biology, 0.5 M in H₂O, MOPS, ≧99.5% (T), MOPS, for luminescence, ≧99.5% (T), MOPS, for molecular biology, ?99.5% (T), magnesium acetate solution, for molecular biology, about 1 M in H₂O, magnesium acetate tetrahydrate, ≧99.0% (KT), magnesium citrate tribasic nonahydrate, ≧98.0% (calc. based on dry substance, KT), magnesium formate solution, 0.5 M in H₂O, magnesium phosphate dibasic trihydrate, ≧98.0% (KT), neutralization solution for the *in situ* hybridization for *in situ* hybridization, for molecular biology, oxalic acid dihydrate, ?99.5% (RT), PIPES, ≧99.5% (T), PIPES, for molecular biology, ≧99.5% (T), phosphate buffered saline, solution (autoclaved), phosphate buffered saline, washing buffer for peroxidase conjugates in Western blotting, 10 times concentrate, piperazine, anhydrous, ≧99.0% (T), potassium D-tartrate monobasic, ≧99.0% (T), potassium acetate solution, for molecular biology, potassium acetate solution, for molecular biology, 5 M in H₂O, potassium acetate solution, for molecular biology, about 1 M in H₂O, potassium acetate, ≧99.0% (NT), potassium acetate, for luminescence, 99.0% (NT), potassium acetate, for molecular biology, ?99.0% (NT), potassium bicarbonate, ?99.5% (T), potassium carbonate, anhydrous, ≧99.0% (T), potassium chloride, ?99.5% (AT), potassium citrate monobasic, ?99.0% (dried material, NT), potassium citrate tribasic solution, 1 M in H2O, potassium formate solution, 14 M in H₂O, potassium formate, ?99.5% (NT), potassium oxalate monohydrate, ≧99.0% (RT), potassium phosphate dibasic, anhydrous, ?99.0% (T), potassium phosphate dibasic, for luminescence, anhydrous, ?99.0% (T), potassium phosphate dibasic, for molecular biology, anhydrous, ≧99.0% (T), potassium phosphate monobasic, anhydrous, ≧99.5% (T), potassium phosphate monobasic, for molecular biology, anhydrous, ≧99.5% (T), potassium phosphate tribasic monohydrate, ?95% (T), potassium phthalate monobasic, ≧99.5% (T), potassium sodium tartrate solution, 1.5 M in H2O, potassium sodium tartrate tetrahydrate, ?99.5% (NT), potassium tetraborate tetrahydrate, ≧99.0% (T), potassium tetraoxalate dihydrate, ≧99.5% (RT), propionic acid solution, 1.0 M in H₂O, STE buffer solution, for molecular biology, pH 7.8, STET buffer solution, for molecular biology, pH 8.0, sodium 5,5-diethylbarbiturate, ?99.5% (NT), sodium acetate solution, for molecular biology, 3 M in H₂O, sodium acetate trihydrate, 99.5% (NT), sodium acetate, anhydrous, ?99.0% (NT), sodium acetate, for luminescence, anhydrous, ≧99.0% (NT), sodium acetate, for molecular biology, anhydrous, ≧99.0% (NT), sodium bicarbonate, ?99.5% (T), sodium bitartrate monohydrate, ≧99.0% (T), sodium carbonate decahydrate, ≧99.5% (T), sodium carbonate, anhydrous, ?99.5% (calc. on dry substance, T), sodium citrate monobasic, anhydrous, ?99.5% (T), sodium citrate tribasic dihydrate, ≧99.0% (NT), sodium citrate tribasic dihydrate, for luminescence, ?99.0% (NT), sodium citrate tribasic dihydrate, for molecular biology, ?99.5% (NT), sodium formate solution, 8 M in H₂O, sodium oxalate, ≧99.5% (RT), sodium phosphate dibasic dihydrate, ≧99.0% (T), sodium phosphate dibasic dihydrate, for luminescence, 99.0% (T), sodium phosphate dibasic dihydrate, for molecular biology, ≧99.0% (T), sodium phosphate dibasic dodecahydrate, ≧99.0% (T), sodium phosphate dibasic solution, 0.5 M in H₂O, sodium phosphate dibasic, anhydrous, ?99.5% (T), sodium phosphate dibasic, for molecular biology, ?99.5% (T), sodium phosphate monobasic dihydrate, ?99.0% (T), sodium phosphate monobasic dihydrate, for molecular biology, ≧99.0% (T), sodium phosphate monobasic monohydrate, for molecular biology, ?99.5% (T), sodium phosphate monobasic solution, 5 M in H₂O, sodium pyrophosphate dibasic, ≧99.0% (T), sodium pyrophosphate tetrabasic decahydrate, ?99.5% (T), sodium tartrate dibasic dihydrate, ≧99.0% (NT), sodium tartrate dibasic solution, 1.5 M in H₂O (colorless solution at 20. degree. C.), sodium tetraborate decahydrate, ≧99.5% (T), TAPS, ≧99.5% (T), TES, ≧99.5% (calc. based on dry substance, T), TM buffer solution, for molecular biology, pH 7.4, TNT buffer solution, for molecular biology, pH 8.0, TRIS Glycine buffer solution, 10. times. concentrate, TRIS acetate-EDTA buffer solution, for molecular biology, TRIS buffered saline, 10. times. concentrate, TRIS glycine SDS buffer solution, for electrophoresis, 10. times. concentrate, TRIS phosphate-EDTA buffer solution, for molecular biology, concentrate, 10. times, concentrate, Tricine, ≧99.5% (NT), Triethanolamine, ≧99.5% (GC), Triethylamine, 99.5% (GC), Triethylammonium acetate buffer, volatile buffer, -1.0 M in H₂O, Triethylammonium phosphate solution, volatile buffer, about 1.0 M in H2O, Trimethylammonium acetate solution, volatile buffer, about 1.0 M in H2O, Trimethylammonium phosphate solution, volatile buffer, about 1 M in H2O, Tris-EDTA buffer solution, for molecular biology, concentrate, 100 times concentrate, Tris-EDTA buffer solution, for molecular biology, pH 7.4, Tris-EDTA buffer solution, for molecular biology, pH 8.0, TRIZMA acetate, ?99.0% (NT), TRIZMA base, ?99.8% (T), TRIZMA base, ≧99.8% (T), TRIZMA base, for luminescence, ≧99.8% (T), TRIZMA base, for molecular biology, ?99.8% (T), TRIZMA carbonate, ≧98.5% (T), TRIZMA hydrochloride buffer solution, for molecular biology, pH 7.2, TRIZMA hydrochloride buffer solution, for molecular biology, pH 7.4, TRIZMA hydrochloride buffer solution, for molecular biology, pH 7.6, TRIZMA hydrochloride buffer solution, for molecular biology, pH 8.0, TRIZMA hydrochloride, ≧99.0% (AT), TRIZMA hydrochloride, for luminescence, ≧99.0% (AT), TRIZMA hydrochloride, for molecular biology, ≧99.0% (AT), and TRIZMA maleate, ≧99.5% (NT).

The composition can comprise one or more emulsifying agents to aid in the formation of the nanoemulsion. Emulsifying agents include compounds that aggregate at the oil/water interface to form a continuous membrane that prevents direct contact between two adjacent droplets. The composition may also further comprise one or more immune modulators. Examples of immune modulators include, but are not limited to, chitosan and glucan. An immune modulator can be present in the composition at any pharmaceutically acceptable amount, e.g., from about 0.001% up to about 10%, and any amount in between, such as about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%, about 0.9%, about 1%, about 2%, about 3%, about 4%, about 5%, about 6%, about 7%, about 8%, about 9%, about 10%, or a range defined by any two of the foregoing values.

The composition may be formulated into pharmaceutical compositions which comprise therapeutically effective amounts of the nanoemulsion and aeroallergen(s) and a pharmaceutically-acceptable carrier. The choice of carrier will be determined by the practitioner, and a variety of suitable pharmaceutically-acceptable excipients are well known in the art.

In certain embodiments, the nanoemulsion comprises (a) about 3 vol. % to about 15 vol. % (e.g., about 4 vol. %, 5 vol. %, 6 vol. %, 7 vol. %, 8 vol. %, 9 vol. %, 10 vol. %, 11 vol. %, 12 vol. %, 13 vol. %, or 14 vol. %) of a poloxamer surfactant or polysorbate surfactant; (b) about 3 vol. % to about 15 vol. % (e.g., about 4 vol. %, 5 vol. %, 6 vol. %, 7 vol. %, 8 vol. %, 9 vol. %, 10 vol. %, 11 vol. %, 12 vol. %, 13 vol. %, or 14 vol. %) of an organic solvent; (c) about 0.5 vol. % to about 1 vol. % (e.g., about 0.6 vol. %, 0.7 vol. %, 0.8 vol. %, or 0.9 vol. %) of a halogen-containing compound; (d) about 3 vol. % to about 85 vol. % (e.g., about 5 vol. %, 10 vol. %, 20 vol. %, 30 vol. %, 40 vol. %, 50 vol. %, 60 vol. %, 70 vol. %, 80 vol. %, or 85 vol. %) of an oil; and (e) about 5 vol. % to about 60 vol. % (e.g., about 10 vol. %, 20 vol. %, 30 vol. %, 40 vol. %, or 50 vol. %) of water. An exemplary nanoemulsion that may be used is designated "W805EC," and the components of which are shown in Table 1. The mean droplet size for the W805EC nanoemulsion is about 400 nm. All of the components of the nanoemulsion are included on the FDA inactive ingredient list for Approved Drug Products.

**Table 1. W805EC Nanoemulsion Formulation**

| **Component** | **Function** |
|---|---|
| Purified water, USP | Aqueous diluent |
| Soybean oil, USP (super refined) | Hydrophobic oil (core) |
| Dehydrated alcohol, USP (anhydrous ethanol) | Organic solvent |
| Polysorbate 80, NF | Surfactant |
| Cetylpyridinium chloride, USP | Emulsifying agent |

Another exemplary nanoemulsion that may be employed in the disclosed composition is designated "60% W805EC," the components of which are set forth in Table 2.

**Table 2. 60% W805EC Formulation**

| **Component** | **Amount (w/w %)** |
|---|---|
| Purified water, USP | 54.10% |
| Soybean oil, USP (super refined) | 37.67% |
| Dehydrated alcohol, USP (anhydrous ethanol) | 4.04% |
| Polysorbate 80, NF | 3.55% |
| Cetylpyridinium chloride, USP | 0.64% |

### Methods of Preparing Nanoemulsions

Generally, nanoemulsions encompassed by the present disclosure are formed by emulsification of an oil, purified water, nonionic detergent, organic solvent, and surfactant (e.g., a cationic surfactant). In this regard, the nanoemulsion may be formed using classic emulsion forming techniques, such as those described in U.S. Patent 7,767,216. In an exemplary method, the oil is mixed with the aqueous phase under relatively high shear forces (e.g., using high hydraulic and mechanical forces) to obtain a nanoemulsion comprising oil droplets having an average diameter of less than about 1000 nm. Some embodiments of the present disclosure employ a nanoemulsion having an oil phase comprising an alcohol such as ethanol. The oil and aqueous phases can be blended using any apparatus capable of producing shear forces sufficient to form an emulsion, such as French presses or high shear mixers (e.g., FDA approved high shear mixers are available, for example, from Admix, Inc., Manchester, N.H.). Methods of producing such emulsions are described in U.S. Patents 5,103,497 and 4,895,452.

In one embodiment, the nanoemulsion may comprise droplets of an oily discontinuous phase dispersed in an aqueous continuous phase, such as water or phosphate buffered saline (PBS). The nanoemulsion can be produced in large quantities and be stable for many months at a broad range of temperatures. The nanoemulsion can have textures ranging from that of a semi-solid cream to that of a thin lotion and can be applied topically by any pharmaceutically acceptable method, e.g., by hand, or nasal drops/spray.

As discussed above, at least a portion of the emulsion may be in the form of lipid structures including, but not limited to, unilamellar, multilamellar, and paucliamellar lipid vesicles, micelles, and lamellar phases.

It will be appreciated that variations of the described nanoemulsions will be useful in the compositions and methods disclosed herein. To determine if a candidate nanoemulsion is suitable for use with the present invention, three criteria are analyzed. First, the desired ingredients are prepared using the methods described herein, to determine if a nanoemulsion can be formed. If a nanoemulsion cannot be formed, the candidate is rejected. Second, the candidate nanoemulsion should form a stable emulsion. A nanoemulsion is stable if it remains in emulsion form for a sufficient period to allow its intended use. For example, for nanoemulsions that are to be stored, shipped, etc., it may be desired that the nanoemulsion remain in emulsion form for months to years. Typical nanoemulsions that are relatively unstable will lose their form within a day. Third, the candidate nanoemulsion should have efficacy for its intended use. For example, the emulsions of the invention should maintain (e.g., not decrease or diminish) and/or enhance the immunogenicity of allergen (e.g., an aeroallergen), or induce a protective immune response to a detectable level.

The nanoemulsion can be provided in many different types of containers and delivery systems. For example, in some embodiments, the nanoemulsion may be provided in a cream or other solid or semi-solid form. Alternatively, the nanoemulsion may be incorporated into hydrogel formulations. The nanoemulsion can be delivered (e.g., to a subject or customers) in any suitable container. Suitable containers can be used that provide one or more single use or multi-use dosages of the nanoemulsion for the desired application. In some embodiments, the nanoemulsions are provided in a suspension or liquid form. Such nanoemulsions can be delivered in any suitable container including spray bottles and any suitable pressurized spray device. Such spray bottles may be suitable for delivering the nanoemulsions intranasally or via inhalation. These nanoemulsion-containing containers can further be packaged with instructions to form kits.

Generally, emulsion compositions disclosed herein will comprise at least 0.001% to 100%, preferably 0.01 to 90%, of emulsion per ml of liquid composition. It is envisioned that the formulations may comprise about 0.001%, about 0.0025%, about 0.005%, about 0.0075%, about 0.01 %, about 0.025%, about 0.05%, about 0.075%, about 0. 1 %, about 0.25%, about 0.5%, about 1.0%, about 2.5%, about 5%, about 7.5%, about 10%, about 12.5%, about 15%, about 20%, about 25%, about 30%, about 35%, about 40%, about 50%, about 55%, about 60%, about 65%, about 70%, about 75%, about 80%, about 85%, about 90%, about 95% or about 100% of emulsion per ml of liquid composition. It should be understood that a range between any two figures listed above is specifically contemplated to be encompassed within the metes and bounds of a composition of the disclosure.

In some embodiments, a nanoemulsion composition is formulated to comprise between 0.1 and 500 µg of antigen (e.g., aeroallergen). For example, the nanoemulsion composition may contain between 0.5 µg and 50 µg (e.g., about 1 µg, about 5 µg, about 10 µg, about 20 µg, about 30 µg, or about 40 µg) of antigen, between 50 µg and 100 µg (e.g., about 60 µg, about 70 µg about 80 µg, or about 90 µg) of antigen, 100 µg or more (e.g., about 200 µg, about 300 µg, or about 400 µg) of antigen, or a range defined by any two of the foregoing values. However, the present invention is not limited to this amount of antigen. For example, in some embodiments, more than 500 µg (e.g., 600 µg, 700 µg, 800 µg, 900 µg, 1 mg, or more of antigen (e.g., aeroallergen) is present in nanoemulsion disclosed herein (e.g., for use in administration to a subject). In some embodiments, less than 0.1 µg of aeroallergen (e.g., 900 nanograms (ng), 800 ng, 700 ng, 600 ng, 500 ng, 400 ng, 300 ng, or less) is present in nanoemulsion disclosed herein for administration to a subject. In other embodiments, more than one type of aeroallergen is present in a nanoemulsion disclosed here.

### Treatment Methods

The disclosure also provides a method of treating aeroallergen induced inflammation (e.g., airway inflammation) in a subject, which comprises administering an effective amount of the above-described nanoemulsion composition to a subject in need thereof, whereupon the aeroallergen induced inflammation (e.g., airway inflammation) in the subject is treated. As used herein, the terms "treatment," "treating," and the like refer to obtaining a desired pharmacologic and/or physiologic effect. Preferably, the effect is therapeutic, i.e., the effect partially or completely cures a disease and/or adverse symptom attributable to the disease. To this end, the inventive method comprises administering a "therapeutically effective amount" of the composition. A "therapeutically effective amount" refers to an amount effective, at dosages and for periods of time necessary, to achieve a desired therapeutic result. The therapeutically effective amount may vary according to factors such as the disease state, age, sex, and weight of the individual, and the ability of the composition to elicit a desired response in the individual. For example, a therapeutically effective amount of the composition of the invention is an amount which decreases aeroallergen induced inflammation (e.g., airway inflammation) or other adverse allergic condition in a human.

Alternatively, the pharmacologic and/or physiologic effect may be prophylactic, i.e., the effect completely or partially prevents a disease or symptom thereof. In this respect, the inventive method comprises administering a "prophylactically effective amount" of the composition. A "prophylactically effective amount" refers to an amount effective, at dosages and for periods of time necessary, to achieve a desired prophylactic result (e.g., prevention of disease onset).

The terms "allergic airway inflammation," "allergen-induced airway inflammation," "aeroallergen induced inflammation," "allergic airway disease," and "respiratory allergy" are used interchangeably herein and generally refer to a condition which results from a predominant T helper 2 (Th2) type response that develops after an initial innocuous inhaled or cutaneous exposure to allergen that causes sensitization to any subsequent allergen contact (Corry et al., Mol. Med., 4: 344-55 (1998); Hogan et al., J. Immunol., 161: 1501-1509 (1998); and Sugita et al., Intern. Med., 42: 636-643, (2003)). Allergic airway inflammation involves an increase in airway eosinophils, basophils and, less consistently, neutrophils. These responses are mediated by the trafficking and activation of myeloid dendritic cells into the airways, probably as a result of the release of epithelial cell-derived thymic stromal lymphopoietin, and the release of pro-inflammatory cytokines from type 2 helper T-cells.

The immune response that occurs upon subsequent exposure to the allergen involves two phases, an early phase and a late phase. The early phase occurs within minutes of allergen exposure and is characterized by a plurality of adverse conditions including, but not limited to, sudden inability to breathe, constriction of the airways, coughing, wheezing, and mucus production. This response is initiated when allergen cross-links FcεR-bound allergen-specific IgE on the surface of mast cells, which induces downstream signaling events that lead to secretion of inflammatory mediators (Kalesnikoff and Galli, Nat. Immunol., 9: 1215-23 (2008); and Walsh et al., Discov. Med., 9(47): 357-62 (2010)). The late phase of an allergic asthma response takes place a few hours after the initial allergen challenge and usually resolves within one to two days. Adverse conditions of the late phase include, but are not limited to, airway narrowing, mucus hypersecretion, and tissue eosinophilia and chronic persistence of this phase of the disease leads to long term remodeling of the lung (Sugita et al., *supra;* Sokol et al., Nat. Immunol., 9: 310-318 (2008)).

Allergic airway inflammation is associated with a variety of different diseases or conditions, including, for example, allergic rhinitis (AR), non-allergic asthma, allergic asthma, chronic rhinosinusitis (CRS), some food allergies, and some drug allergies. The disclosed compositions and methods can be used to treat any disease or condition associated with aeroallergen induced inflammation (e.g., airway inflammation). In some embodiments, the method may be used to treat asthma (aeroallergen induced asthma). Asthma is an inflammatory airway disease characterized by the presence of cells such as eosinophils, mast cells, basophils, and CD25+ T lymphocytes in the airway walls. Chemokines attract cells to the site of inflammation and cytokines activate them, resulting in inflammation and damage to the mucosa. When asthma becomes chronic, secondary changes occur, such as thickening of basement membrane and fibrosis. In general, asthma can be subdivided into three forms: (i) extrinsic/allergic asthma, which is clearly caused by an allergen, (ii) intrinsic/non-allergic asthma, which is not linked allergen exposure, and (iii) a mixed form. In allergic/extrinsic asthma, the initiation event of airway inflammation is an immunological reaction to allergen. Continued exposure to allergen results in chronic inflammation. Allergic asthma affects about 70% of all asthma patients (Romanet-Manent et al., Allergy, 57: 607-613 (2002)). In contrast, 10% to 33% of all asthma patients suffer from "non-allergic asthma," which has a later onset than allergic asthma, a more severe clinical course in adults, and is significantly associated with nasal polyps in combination with aspirin idiosyncrasy (Humbert et al., Immunol Today, 20: 528-533 (1999); Novak, N. and T. Beiber, Journal of Allergy and Clinical Immunology, 112 (2): 252-262, (2003)).

While the compositions ideally comprises one or more aeroallergens, compositions comprising non-airborne allergens are also within the scope of this disclosure. For example, the composition may comprise one or more allergens that induce any atopic disease. The term "atopic," as used herein, refers to a hereditary predisposition toward developing certain hypersensitivity reactions (e.g., eczema (atopic dermatitis), hay fever (allergic rhinitis), and allergy-induced asthma (allergic asthma)), which are typically mediated by excessive IgE production. Such disorders include, but are not limited to, allergic inflammation of the skin, lungs, and gastrointestinal tract, atopic dermatitis (also known as atopic eczema), fibrosis (e.g., idiopathic pulmonary fibrosis, scleroderma, kidney fibrosis, and scarring), some food allergies (e.g., allergies to peanuts, eggs, dairy, shellfish, tree nuts, etc.), and other allergies.

The nanoemulsion composition can be administered to a mammal using standard administration techniques, including oral, intravenous, intraperitoneal, subcutaneous, pulmonary, transdermal, intramuscular, intranasal, buccal, sublingual, or suppository administration. The composition preferably is suitable for intranasal administration. Intranasal administration includes administration via the nose, either with or without concomitant inhalation during administration. Such administration is typically through contact by the with the nasal mucosa, nasal turbinates or sinus cavity. Such administration may also include contact with the oral mucosa, bronchial mucosa, and other epithelia. The composition may be applied in a single administration or in multiple administrations.

Administration of the composition comprising a nanoemulsion in combination with one or more aeroallergens desirably inhibits the initiation or progression of aeroallergen induced inflammation (e.g., airway inflammation (e.g., chronic allergic asthma)). Thus, the composition desirably is administered after exposure (or after suspected exposure or prior to impending exposure) to an allergen (e.g., an aeroallergen) to which the subject is hypersensitive. For example, the composition may be administered at least once between 0 to 30 days, between 0 to 20 days, between 0 to 15 days, or between 0 to 7 days, after the subject has been exposed to the allergen to which the subject is hypersensitive. In another aspect, the composition may be administered daily for a specified time. For example, the composition may be administered daily for at least one week, at least one month, at least 3 months, 6 months, a year, or longer.

In some embodiments, the composition may be administered in a regimen that includes at least 1, 2, 3, 4, 5, 6, 7, 8, 9, 10, or more cycles of daily treatment. A cycle includes: (a) a period during which the composition is administered daily (e.g., 1-30 days), followed by (b) a rest period of at least one day (e.g., at least one week, 2 weeks, 3 weeks, a month, or more) in which the composition is not administered. The number of days of administration and rest can be the same or different within a cycle. Likewise, two or more consecutive cycles can have the same or a different duration.

Administration of the composition described herein desirably results in the reduction or inhibition of the expression of Th2 type cytokines in the subject. Thus, in some embodiments, the disclosed compositions may be used to modulate (e.g., reduce or skew away from) Th2 immune responses (characterized by robust expression of Th2 cytokines (IL-4, IL-5, and IL-13) and IgG1) and toward a balanced Th1/Th2 response (characterized by reduced IgE and Th2 response and increased IFN-gamma, TNF-alpha, IgG2a, IgG2b, IgA, IL-10, and IL-17) as a therapeutic for allergic disease, inflammatory disease, or any other disease associated with Th2 immunity.

The following examples further illustrate the invention but, of course, should not be construed as in any way limiting its scope.

### EXAMPLES

The following materials and methods were used in the experiments described below.

**Antigen and adjuvants.** Endotoxin-free ovalbumin (ova) was purchased from Lionex (Branschweig, Germany). The cockroach allergen (CRA) was clinical grade, skin test CRA (Hollister Stier, Toronto, Canada) that was purified by centrifugation using AM1CON^{®} Ultra-15 Centrifugal Filter Unit with ULTRACEL^{®}-3 membrane, 3000 MWCO to obtain endotoxin free CRA. Nanoemulsion adjuvant (NE) was produced by a high-speed emulsification of ultra-pure soybean oil with cetyl pyridinium chloride, Tween 80 and ethanol in water, resulting in NE droplets with an average 350-400 nm diameter (Makidon et al., PLoS One, 3(8): e2954 (2008); and Myc et al., Vaccine, 21(25-26): 3801-14 (2003)). Aluminum hydroxide (alum, alhydrogel) was purchased from InvivoGen (San Diego, CA). Incomplete Freund's Adjuvant (IFA) was purchased from Sigma-Aldrich (St. Louis, MO). The absence of endotoxin in all reagents was confirmed using limulus assay.

**Ovalbumin allergic asthma model.** Specific pathogen-free BALB/c mice (females 4-5 weeks old) were purchased from Jackson Laboratory and immunized as per the schedule shown in FIG. 8A. For all immunizations, mice were anesthetized under isoflurane anesthesia using the IMPAC6 precision vaporizer. Allergic sensitization was induced with intraperitoneal immunizations (i.p.) of 20 µg ova adsorbed on 2 mg alum (Daubeuf F, Frossard N., Curr Protoc Mouse Biol., 3(1): 31-7 (2013)). Intranasal (i.n.) immunizations were administered as 12 µl (6 µl /nare) of a formulation containing 20 µg of ova mixed with 20% NE (O'Konek et al., J Allergy Clin Immunol., 141(6): 2121-31 (2018); and O'Konek et al., Allergy, 75(4): 872-881 (2020). doi: 10.1111/all.14064). Ova mixed with PBS alone served as a control. Mice were challenged intratracheally with 100 µg of ova on three alternating days during week 16. Mice were sacrificed two days after the third challenge. All animal procedures were performed according to the University of Michigan Institutional Animal Care and Use Committee and the National Institutes of Health Guide for the Care and Use of Laboratory Animals.

**Lung histology.** At the time of sacrifice, lungs were perfused with 4% formaldehyde for fixation. After fixation, lungs were embedded in paraffin, sliced transversally into 5-µm thick sections, and stained with periodic acid-Schiff (PAS) to detect cellular infiltration and mucus production. The lung sections were scored for inflammation using the following scoring system: 0, absent; 1, minimal; 2, slight; 3, moderate; and 4, severe (de Almeida Nagata et al., Am J Pathol., 184(6): 1807-18 (2014)). Total number of airways were counted and scored as mucus positive or mucus negative to determine the percentage of airways producing mucus.

**Analysis of cytokine expression.** To assess allergen-specific recall responses, red blood cell-depleted splenocytes or lymphocytes isolated from cervical lymph nodes were cultured *ex vivo* ± ova (20 µg/ml). After 72 hours, cytokine secretion was measured in cell culture supernatants using Luminex Multiplex detection system (Millipore, Billerica, MA). To quantify cytokines in lung tissue, lungs were isolated one day after the final ova challenge and homogenized in 350 µL of T-PER tissue extraction buffer (Thermo Scientific), and frozen at - 80°C. Samples were subjected to an additional freeze/thaw cycle, and then centrifuged at 10,000xg for 5 minutes at 4°C to remove debris. Cytokines in lung supernatants were analyzed using a Luminex Multiplex kit. ELISpot assays were run using kits from Mabtech according to manufacturer's instructions. Briefly, sterile 96-well multiscreen filter plates with PVDF membrane (Millipore) were coated overnight with anti-IΓN-γ, IL-5, or IL-17 capture antibodies, blocked with 5% fetal bovine serum, and cells were added at 500,000 to 1,000,000 cells per well. Cells were cultured ± ova (20 µg/ml) for 40 hours and cytokine secreting cells were detected by incubation with biotinylated antibodies to the respective cytokines followed by streptavidin-alkaline phosphatase. Spots were developed by addition of BCIPINBT substrate and counted using an AID ELISpot reader system.

**Mouse chronic CRA asthma model.** Mice were sensitized by i.p. and subcutaneous (s.c.) injection of 500 protein nitrogen units (pnu) of CRA mixed 1:1 in IFA (Sigma-Aldrich, St. Louis, MO). Next, mice were challenged intranasally with 150 pnu of CRA on days 14, 18, and 22 after initial CRA sensitization to localize the response to the lung (Fonseca et al., Mucosal Immunol., 12(2): 445-56 (2019)). Mice were immunized on days 28, 56, and 84 with a formulation containing 20 µg of CRA mixed with 20% NE (12 µl/mouse; 6 µl /nare) or CRA mixed with PBS as a control. Mice were challenged by intratracheal injection with 500 pnu CRA on days 98 and 102. Mice were sacrificed, and samples were taken one day after the last allergen challenge.

**Quantitative RT-PCR.** Lung tissue was homogenized and RNA was extracted using TRIzol reagent (Invitrogen, Carlsbad, CA). mRNA concentration was quantified by NanoDrop^{™}, followed by cDNA synthesis using a High Capacity cDNA Reverse Transcription kit (Applied Biosystems, Foster City, CA). Real-time quantitative PCR was performed using Power SYBR green PCR master mix (Applied Biosystems, Foster City, CA). Gene expression was quantified by ΔΔCt analysis and normalized to GAPDH levels within individual samples.

**Measurement of airway hyperreactivity (AHR).** AHR was assessed using a mouse plethysmograph specifically designed for low tidal volumes (Buxco Research Systems), as described previously (Lindell et al., PLoS One, 6(7): e21823 (2011)). Briefly, mice were anesthetized with sodium pentobarbital, intubated and ventilated at a volume of 200 µl with a frequency of 120 breaths/minute. The plethysmograph was sealed, so changes in lung volume were represented by changed box pressure. Airway resistance was measured in by assessing tracheal pressure and comparing to the corresponding box pressure changes. Baseline levels were determined, and mice were challenged via tail vein with 0.35 mg/kg of methacholine. The peak airway resistance was recorded to quantify AHR.

**Measurement of serum antibodies.** Blood was collected at the end of the study, and sera were harvested by centrifugation. Ova- and CRA-specific IgG1, IgG2a and IgE antibodies were determined by ELISA in serially diluted serum, using ova- and CRA-coated 96-well plates and alkaline phosphatase conjugated detection antibodies as described previously (Makidon et al., *supra*).

**Flow cytometry.** The animals' lungs were removed and digested with 1 mg/ml collagenase A (Roche, Indianapolis, IN) and 20 U/ml DNaseI (Sigma, St. Louis, MO) in RPMI 1640 containing 10% FCS. Single cell suspensions were achieved by dispersion through an 18-gauge needle and filtration through 100-µm cell strainer. Cells were resuspended in PBS and stained by flow cytometry. All antibodies used for flow cytometry were purchased from BioLegend unless otherwise noted. Fc receptors were blocked with purified anti-CD16/32 and surface markers were identified using antibodies against the following antigens: B220, CD3, CD4, CD1 1b, CD25, CD45, CD90, Gr-1, ST2 and Ter119. Cells were fixed, permeablized, and labeled for intracellular Foxp3 (eBioscience) and GATA3 (eBioscience). Cell types were defined as follows: Treg: CD4⁺CD25⁺Foxp3⁺. Activated Th cells: CD4⁺CD69⁺. ILC2: Lin⁻CD45⁺ CD90⁺ST2⁺GATA3^{+.} For innate lymphoid cell staining, lineage markers were CD3, CD11b, 195 B220, Gr-1, and TER119. Samples were acquired on a NovoCyte flow cytometer (Acea Biosciences). Data were analyzed using FlowJo (Treestar).

**Statistics.** Statistical comparisons were assessed by the Mann-Whitney test using GraphPad Prism version 8 (GraphPad Software). The p value < 0.05 was considered significant.

### EXAMPLE 1

This example describes a model of chronic allergen exposure using cockroach allergen (CRA) sensitization and challenge.

A mouse model of chronic allergen-induced airway remodeling has been developed that is accompanied by intense peribronchial leukocyte recruitment, mucus hypersecretion, development of airway hyperreactivity (AHR), and significant peribronchial and airway thickening (Berlin et al., Lab Invest, 86(6): 557-565 (2006); and Lukacs et al., Journal of Experimental Medicine, 194(4): 551-555 (2001)). It was investigated whether a vaccine targeting cockroach allergen using a nanoemulsion as described herein can alter that ongoing immune response by skewing the responses away from the dominant Th2 cytokine profile. A modified cockroach allergen (CRA) sensitization and challenge protocol was used, which is outlined in FIG. 1. Once sensitized with allergic responses localized to the lungs over a 22 day protocol, animals were given CRA emulsified in the nanoemulsion by intranasal application (8 µl/nare as a mucosal application, which contains 10 µg of CRA). Nanoemulsion with CRA ("CRA-NANO") or without CRA ("NANO") was applied three times four weeks apart at day 28, 56, and 84 days post initial CRA immunization. After an additional 14 days (day 98) the animals were challenged twice by intratracheal administration of CRA four days apart. This model of chronic allergen exposure is steroid resistant to treatment by systemic dexamethasone (3 mg/kg IP) and involves a Th2 dominant response with little or no induction in IL-17 and IFN.

The mice treatment groups are set forth in Table 3.

**Table 3.**

| **Treatment Group** | **Number of Mice (n)** | **Treatment** |
|---|---|---|
| 1 | 6 | Naïve/no treatment |
| 2 | 7 | CRA only |
| 3 | 8 | Nanoemulsion + CRA |
| 3 | 8 | Nanoemulsion only |

Twenty-four hours post final challenge animals were assessed for disease and harvested for analysis. The animals were examined for a number of parameters to identify the changes induced by the immunization/modulation with the nanoemulsion.

### EXAMPLE 2

This example demonstrates that a nanoemulsion composition described herein reduces histopathology and disease-associated parameters in aeroallergen challenged mice described in Example 1.

Examination of the histopathology from the experiments described in Example 1 revealed a visible reduction in overall inflammation and mucus production in the airways of animals given nanoemulsion plus CRA compared to those given CRA without nanoemulsion or nanoemulsion alone (see FIG. 2B). The lungs exhibited a visible reduction in cells in both the peribronchial area as well as the perivascular regions of the tissue. The mRNA of key genes, muc5ac and gob5/clca3, which have been linked to severity of disease and mucus hypersecretion, were examined in the treated groups. As shown in FIG. 3, animals that were treated with the nanoemulsion plus CRA had significant decreases in muc5ac and gob5 mRNA as compared to CRA only and nanoemulsion only treatment groups. These genes are indicative of the severity of the responses and the histopathology shown in FIG. 2B. Expression of IL-17 or IFNγ was not observed in any of the groups examined.

In addition to the changes in histopathology, the animals were also tested for changes in airway hyperreactivity (AHR) using a methacholine challenge by IV tail vein injection (250 µg/kg). As shown in FIG. 4, animals that received the nanoemulsion plus CRA ("CRA Nano") exhibited a significant decrease in AHR compared to those receiving CRA only and nanoemulsion only. Together, these data indicate that a composition comprising a nanoemulsion and the CRA allergen decreases inflammation and mucus hypersecretion and produces physiologic changes in the lungs of mice with severe allergic asthma.

Because the protection conferred by the NE vaccine was associated with changes in Th2 cytokines, it was hypothesized that NE vaccine protected predominantly by altering the allergen-associated cellular inflammation. In this regard, IL-13 is a key Th2 cytokine linked to the severity of disease in allergic asthma, and it was found to be significantly decreased in the lungs of mice that received the NE vaccine before antigen challenge, as shown in FIG. 5A. Lymphocyte populations in the lung that specifically produce different cytokines were evaluated to assess whether the NE vaccine alters their numbers. Specifically, the left lobe of the lung was harvested and dispersed into a single cell suspension using collagenase digestion. After removing debris through low speed centrifugation, the cells were subjected to flow cytometry staining that identified the different cell populations of interest: Treg cells (CD4⁺, CD25⁺, foxp3⁺), activated Th cells (CD4⁺, CD69⁺), and ILC2 cells (Lin⁻, CD45⁺, CD90⁺, ST2⁺, GATA3⁺). FIG. 5 shows that, while there was no difference in Treg cell or CD4⁺, CD69⁺ Th cell numbers in the lung, there was a significant decrease in ILC2 cells in the lungs of the mice treated with nanoemulsion plus CRA. There was no difference in the total CD4 or total CD8 cell populations in the lungs of nanoemulsion-alone-treated vs. CRA-alone-treated mice. These data reflect that in this chronic model of Th2 disease the ILC2 cells were a prominent source of IL-13 that was significantly decreased locally upon intranasal administration of nanoemulsion plus CRA.

The lung draining lymph nodes were also harvested, prepared in a single cell suspension, and rechallenged by CRA with supernatants collected at 48 hours. The data were assessed by multiplex protein assay, which indicated that, while the response to CRA was exclusively a Th2 response, none of the treatment groups were different from one another (see FIG. 6). These latter cytokine activation assays were set up with the same cell numbers from lymph nodes and only assessed whether there was an overall change in the phenotype. The assays did not assess the number of cells in the nodes, whereas the lung data indicated that there was a significant reduction in the intensity and severity. Importantly, compared to other models of allergic lung inflammation (such as ovalbumin and house dust mite), this model did not induce any increase in IL-17 or IFNγ.

A final parameter that was examined was the serum IgE levels in the animals that had undergone the nanoemulsion treatment protocol. The data in FIG. 7 indicate that when animals were treated intranasally with nanoemulsion plus CRA, there was a significant decrease in IgE titer compared to those animals treated with CRA alone, indicating that there was a systemic effect of the local treatment protocol.

### EXAMPLE 3 (for illustrative purpose, not being part of the invention)

This example demonstrates that a nanoemulsion composition described herein protects against airway inflammation in an ova asthma model.

As described previously, BALB/c mice were sensitized with ovalbumin (ova) and aluminum hydroxide (alum) to induce a Th2 allergic phenotype (Brewer et al., J Immunol., 163(12): 6448-54 (1999); and Pichavant et al., Curr Proloc Immunol., Chapter 15: Unit 15 8 (2007)). Animals were then immunized 3 times with either nanoemulsion (NE) adjuvant-ova vaccines or allergen in PBS as a control, as shown in FIG. 8A. Following inhalation challenge with ova, histopathological analyses of lung tissue were performed to characterize the effect of the NE allergy vaccine. As shown in FIG. 8, mice sensitized with ova-alum had significant infiltration of inflammatory cells in the lungs after allergen challenge (p=0.0061 vs ova-PBS group). This inflammation was greatly diminished in mice that received therapeutic ova-NE vaccines, as documented by a significant decrease in cellular infiltrates (p=0.0016). The inflammation in the lungs of the ova-NE immunized mice after antigen challenge was focal in nature and did not disrupt the pulmonary architecture. NE immunization also induced significant reductions in allergen-induced mucus production (FIG. 8D; p=0.0002). Sensitized mice had mucus in approximately 28% of their airways after ova challenge as compared with 8% of the airways in mice receiving the NE immunizations. In the NE-treated mice, the airways that contained mucus had significantly less mucus and fewer mucus-producing cells, suggesting an inhibition of the goblet cell hyperplasia observed in ova-sensitized mice who were not immunized with NE.

### EXAMPLE 4 (for illustrative purpose, not being part of the invention)

This example demonstrates that intranasal immunization with a nanoemulsion composition described herein suppresses acute allergic Th2 cytokine production in the ova model.

To examine the effect of NE adjuvant alterations in the cellular immune response to ova, cytokine production was evaluated by ELISpot (FIG. 9A) and Luminex (FIG. 9B and FIG. 9C) to quantify, respectively, both the number of cytokine-producing cells and the amount of cytokine secreted. Upon *ex vivo* ova stimulation with allergen, cells from ova-alum sensitized mice produced predominantly IL-5 vs. IFN-γ (FIG. 9A). This changed in animals immunized with NE-ova, where IFN-γ and IL-17 cells predominated (FIG. 9A). In addition, there were no significant differences in the cellular profile between sensitized mice that received ova-NE immunization and mice that were immunized with ova-NE alone (FIG. 9A). This suggested that the NE-ova immunizations could redirect the phenotype of the ova T cell response. Similarly, cervical lymph node lymphocytes from mice that received subsequent ova-NE immunizations produced significantly more Th1 cytokines, including IFN-γ and IL-2, and significantly less Th2 cytokines, such as IL-5 and IL-13 (FIG. 9B). Additionally, ova-NE treatment significantly increased both IL-17 and IL-10 production (p=0 0001 and 0.0047, respectively). Similar patterns were observed in cultured splenocytes from these animals, with more dramatic reductions in IL-5 and IL-13 (FIG. 9C).

The results of this example indicate that the ova-NE nasal immunizations altered both local and systemic immune responses.

### EXAMPLE 5

This example demonstrates that the nanoemulsion adjuvant vaccine induced reductions in pathology in both acute and chronic asthma models despite significant levels of allergen-specific IgE.

Humoral immunity to the eliciting allergen was characterized in both the CRA and ova asthma models. In both models, allergen-specific IgE was not detectable before sensitization. In the ova model, titers of anti-ova IgE titers increased dramatically after alum immunization, to 10⁴, and subsequent immunization with the NE vaccine decreased IgE significantly (p=0.0139; 10-fold, FIG. 10A). In addition, while IgG2a was significantly increased by the NE vaccine, IgG1 titers were not changed (FIG. 10A). NE vaccination did not significantly alter the CRA-specific IgE, IgG1 or IgG2a in the chronic asthma model, where sensitized mice have high titers of all three antibody classes. Therefore, the reduction of inflammation and airway hyperreactivity induced by NE in both models occurred with minimal modulation of the humoral immune response.

### EXAMPLE 6

This example demonstrates that nanoemulsion adjuvant-immunized mice have reduced alarmins in the lungs following allergen challenge.

The activation and proliferation of ILC2 cells depends upon alarmin cytokines, including IL-25 and IL-33 (Claudio et al., J Immunol., 195(8): 3525-9 (2015); Barlow et al., J Allergy Clin Immunol., 129(1):191-8 e1-4 (2012); Halim et al., Nat Immunol., 17(1): 57-64 (2016). doi: 10.1038/ni.3294; and Mikhak et al., J Allergy Clin Immunol., 123(1): 67-73 e3 (2009). It was hypothesized that reduced lung ILC2s in NE-immunized mice may be due to changes in the production of these cytokines. Cytokine levels were quantified in lungs isolated following allergen challenge. Both IL-25 and IL-33 were significantly reduced in the lungs of mice that received the NE vaccine prior to allergen challenge (FIG. 11).

These data suggest that intranasal administration of NE-adjuvanted allergen reduces lung ILC2 cells through suppression of the alarmins IL-25 and IL-33.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention.

## Claims

1. A composition comprising a nanoemulsion and one or more aeroallergens wherein the aeroallergen is a cockroach allergen, and wherein the nanoemulsion comprises:
(a) a poloxamer surfactant or polysorbate surfactant;
(b) an organic solvent;
(c) a halogen-containing compound;
(d) oil, and
(e) water.

2. The composition of claim 1, wherein the nanoemulsion comprises:
a) about 3 vol. % to about 15 vol. % of a poloxamer surfactant or polysorbate surfactant;
b) about 3 vol. % to about 15 vol. % of an organic solvent;
c) about 0.5 vol. % to about 1 vol. % of a halogen-containing compound;
d) about 3 vol. % to about 85 vol. % of an oil; and
e) about 5 vol. % to about 60 vol. % of water.

3. The composition of any one of claims 1-2 for use in treatment of an allergic airway inflammation.

4. The composition of any one of claims 1-2 for the use of claim 3, wherein the subject is a human.

5. The composition of any one of claims 1-2 for the use of claim 4, wherein the allergic airway inflammation is selected from the group consisting of asthma and allergic rhinitis.

6. The composition of any one of claims 1-2 for the use of any one of claims 3-5, wherein the use results in reduction of the expression of Th2 type cytokines.

7. The composition of any one of claims 1-2 for the use of any one of claims 3-6, wherein the composition is administered intranasally.

## Patentansprüche

1. Zusammensetzung, umfassend eine Nanoemulsion und ein oder mehrere Aeroallergene, wobei es sich bei dem Aeroallergen um ein Schabenallergen handelt und wobei die Nanoemulsion Folgendes umfasst:
(a) ein Poloxamertensid oder Polysorbattensid;
(b) ein organisches Lösungsmittel;
(c) eine halogenhaltige Verbindung;
(d) Öl und
(e) Wasser.

2. Zusammensetzung nach Anspruch 1, wobei die Nanoemulsion Folgendes umfasst:
a) etwa 3 Vol.-% bis etwa 15 Vol.-% eines Poloxamertensids oder Polysorbattensids;
b) etwa 3 Vol.-% bis etwa 15 Vol.-% eines organischen Lösungsmittels;
c) etwa 0,5 Vol.-% bis etwa 1 Vol.-% einer halogenhaltigen Verbindung;
d) etwa 3 Vol.-% bis etwa 85 Vol.-% eines Öls; und
e) etwa 5 Vol.-% bis etwa 60 Vol.-% Wasser.

3. Zusammensetzung nach einem der Ansprüche 1-2 zur Verwendung bei der Behandlung einer allergischen Atemwegsentzündung.

4. Zusammensetzung nach einem der Ansprüche 1-2 zur Verwendung nach Anspruch 3, wobei es sich bei dem Patienten um einen Menschen handelt.

5. Zusammensetzung nach einem der Ansprüche 1-2 zur Verwendung nach Anspruch 4, wobei die allergische Atemwegsentzündung aus der Gruppe bestehend aus Asthma und allergischer Rhinitis ausgewählt ist.

6. Zusammensetzung nach einem der Ansprüche 1-2 zur Verwendung nach einem der Ansprüche 3-5, wobei die Verwendung zu einer Verringerung der Expression von Cytokinen des Th2-Typs führt.

7. Zusammensetzung nach einem der Ansprüche 1-2 zur Verwendung nach einem der Ansprüche 3-6, wobei die Zusammensetzung intranasal verabreicht wird.

## Revendications

1. Composition comprenant une nanoémulsion et un ou plusieurs aéroallergènes, où l'aéroallergène est un allergène de blatte, et où la nanoémulsion comprend :
(a) un tensioactif de poloxamère ou un tensioactif de polysorbate ;
(b) un solvant organique ;
(c) un composé contenant un halogène ;
(d) de l'huile, et
(e) de l'eau.

2. Composition selon la revendication 1, dans laquelle la nanoémulsion comprend :
a) environ 3% en volume à environ 15% en volume d'un tensioactif de poloxamère ou d'un tensioactif de polysorbate ;
b) environ 3% en volume à environ 15% en volume d'un solvant organique ;
c) environ 0,5% en volume à environ 1% en volume d'un composé contenant un halogène ;
d) environ 3% en volume à environ 85% en volume d'une huile ; et
e) environ 5% en volume à environ 60% en volume d'eau.

3. Composition selon l'une quelconque des revendications 1 et 2 pour une utilisation dans le traitement d'une inflammation allergique des voies respiratoires.

4. Composition selon l'une quelconque des revendications 1 et 2 pour l'utilisation selon la revendication 3, dans laquelle le sujet est un être humain.

5. Composition selon l'une quelconque des revendications 1 et 2 pour l'utilisation selon la revendication 4, dans laquelle l'inflammation allergique des voies respiratoires est choisie dans le groupe constitué par l'asthme et la rhinite allergique.

6. Composition selon l'une quelconque des revendications 1 et 2 pour l'utilisation selon l'une quelconque des revendications 3 à 5, dans laquelle l'utilisation entraîne une réduction de l'expression des cytokines de type Th2.

7. Composition selon l'une quelconque des revendications 1 et 2 pour l'utilisation selon l'une quelconque des revendications 3 à 6, dans laquelle la composition est administrée par voie intranasale.
